# EUROPEAN PATENT APPLICATION

(11) **EP 2 921 228 A1**
(43) Date of publication of application: **23.09.2015**
(21) Application number: 13854981.1
(22) Date of filing: 14.11.2013
(51) Int. Cl.: B01J 31/22, C07B 53/00, C07C 29/56, C07C 35/17, C07B 61/00, C07C 39/06, C07C 39/17, C07C 43/23, C07F 5/06

(54) **METHOD FOR PRODUCING OPTICALLY ACTIVE ISOPULEGOL AND OPTICALLY ACTIVE MENTHOL**

(30) Priority: 15.11.2012 JP 2012251301; 06.03.2013 JP 2013044065
(71) Applicant: Takasago International Corporation, Tokyo 144-8721 (JP)
(72) Inventor: ITOH, Hisanori, Hiratsuka-shi, Kanagawa 254-0073 (JP); HORI, Yoji, Hiratsuka-shi, Kanagawa 254-0073 (JP); MATSUDA, Hiroyuki, Hiratsuka-shi, Kanagawa 254-0073 (JP); MATSUMURA, Kazuhiko, Hiratsuka-shi, Kanagawa 254-0073 (JP); MATSUMOTO, Takaji, Hiratsuka-shi, Kanagawa 254-0073 (JP)
(74) Representative: Takeuchi, Maya
(86) International application number: PCT/JP2013/080803
(87) International publication number: WO 2014/077323

(57) **Abstract**

The purpose of the present invention is to provide a method for producing optically active isopulegol and optically active menthol which makes it possible to reduce production expenses and minimize environment-polluting waste. The present invention makes it possible to obtain optically active isopulegol with high yield and high selectivity by: obtaining optically active citronellal by asymmetrically isomerizing geraniol and/or nerol using a specific optically active ruthenium catalyst, and performing a selective ring-closing reaction of the optically active citronellal by using an aluminum catalyst obtained by mixing an aluminum alkyl compound and a specific hydroxy compound.

## Description

### Technical Field

The present invention relates to a method for producing an isopulegol and a menthol especially in an economically advantageous short process. In particular, the present invention relates to a method for producing an optically active isopulegol and an optically active menthol. Concretely, the present invention relates to a method in which (E)-3,7-dimethyl-2,6-octadien-1-ol (hereinafter expressed as geraniol) or (Z)-3,7-dimethyl-2,6-octadien-1-ol (hereinafter expressed as nerol) is asymmetrically isomerized, thereby giving an optically active citronellal, and the resultant optically active citronellal is cyclized using an aluminum catalyst, thereby giving an optically active isopulegol, and further, the resultant optically active isopulegol is, without being purified or after crystallized to increase the purity thereof, hydrogenated, thereby giving an optically active menthol.

### Background Art

Menthol is one of most important cooling substances, most of it still being supplied from natural resources. However, the production of natural menthol fairly depends on weather of that year, and the supply thereof is unstable, and therefore a part of it is necessary to rely on synthetics. For producing 1-menthol ((1R,2S,5R)-menthol) on an industrial scale, the economic potential and the efficiency of the synthesis method must be maximized. For this, in particular, the problem is how to synthesize an inexpensive and high-purity 1-menthol from an inexpensive achiral starting material.

L-menthol may be synthesized according to two routes. One is, for example, such that a stereo mixture of menthol obtained through hydrogenation of thymol is esterified, followed by subjecting to optical resolution (through crystallization or enzymatic resolution), thereby giving the compound (Patent Documents 1 and 2).

Regarding the other, S. Akutagawa describes synthesis of 1-menthol according to a key step of asymmetric isomerization from allylamine to enamine using rhodium-BINAP (BINAP = 2,2'-bis(diphenylphosphino)-1,1'-binaphthyl) as a catalyst (Non-Patent Document 1).

A method of asymmetric synthesis of 1-menthol through asymmetric hydrogenation of piperitenone as an important stage is disclosed (Patent Document 3).

Also disclosed is a method for producing an optically active menthol from geraniol, nerol or a mixture of geraniol and nerol, according to a method that includes a) asymmetrically hydrogenating geraniol, nerol or a mixture of geraniol and nerol, thereby giving an optically active citronellol, b) oxidizing the resultant optically active citronellol, thereby giving an optically active citronellal, c) cyclizing the resultant optically active citronellal, thereby giving a mixture containing an optically active isopulegol, then taking out the optically active isopulegol from the resultant mixture, and thereafter d) hydrogenating this, thereby giving an optically active menthol (Patent Document 4).

Further, disclosed is a method for producing an optically active menthol from geranial, neral or a mixture of geranial and neral, according to a method that includes a) precision-distilling citral (mixture of geranial and neral), thereby giving geranial and neral, b) asymmetrically hydrogenating the geranial or neral, thereby giving an optically active citronellal, c) cyclizing the resultant optically active citronellal, thereby giving a mixture containing an optically active isopulegol and taking out the optically active isopulegol from the resultant mixture, and thereafter d) hydrogenating it, thereby giving an optically active menthol (Patent Document 5).

In addition, also disclosed is a method for producing an optically active menthol from at least one of geranial and neral, according to a method that includes a) preparing an optically active citronellal through asymmetric hydrogenation of at least one of geranial and neral, b) cyclizing the resultant optically active citronellal with an acidic catalyst, thereby giving a mixture containing an optically active isopulegol and taking out the optically active isopulegol from the resultant mixture, and thereafter c) hydrogenating it, thereby producing an optically active menthol (Patent Document 6).

An important intermediate for synthesizing racemic and optically active menthol is isopulegol, and generally this is produced through cyclization of citronellal by oxo-ene reaction in the presence of a Lewis acid catalyst, and is, in general, obtained as a mixture of four types of diastereomers, that is, isopulegol, iso-isopulegol, neo-isopulegol and neoiso-isopulegol. Of those, as a method of obtaining the important isopulegol with high selectivity, a method of cyclizing citronellal with an aluminum catalyst is disclosed (Patent Documents 7 to 12).

As a means of synthesizing an optically active citronellal that is a starting material for synthesis of isopulegol and menthol, from geraniol or nerol, there is a method of utilizing asymmetric isomerization of an allyl alcohol containing geraniol and nerol.

Heretofore, as a method of asymmetrically isomerizing an allyl alcohol to give an optically active aldehyde, there are known methods of using a transition metal complex. However, in these methods, the catalyst activity is low, and the optical purity of the resultant optically active aldehyde is not sufficiently satisfactory (Non-Patent Documents 2 to 5).

Further, there are known methods of using a complex of transition metal such as rhodium or ruthenium. However, these methods require high substance specificity and therefore could not be said to be versatile methods (Non-Patent Documents 6 to 17).

Recently, a catalytic reaction with high versatility for producing an optically active aldehyde, which enables highly-selective and high-yield production has been found (Non-Patent Document 18).

### Citation List

### Patent Literature

Patent Document 1: EP 0743295
Patent Document 2: EP 0563611
Patent Document 3: US 6342644
Patent Document 4: JP-T 2008-521763 (the term "JP-T" as used herein means a published Japanese translation of a PCT patent application)
Patent Document 5: WO 2009/068444
Patent Document 6: WO 2012/074075
Patent Document 7: JP-A 2002-212121
Patent Document 8: JP-T 2008-538101
Patent Document 9: WO 2009/144906
Patent Document 10: WO 2010/071227
Patent Document 11: WO 2010/071231
Patent Document 12: JP-A 2011-246366

### Non-Patent Literature

Non-Patent Document 1: Topics in Catalysis 4 (1997) pp. 271-274
Non-Patent Document 2: J. Organomet. Chem., 2002, 650, 1-24
Non-Patent Document 3: Chem. Rev., 2003, 103, 27-51
Non-Patent Document 4: Modern Rhodium-Catalyzed Organic Reactions (Ed.: P.A. Evans), Wiley-VCH, Weinheim, 2005, pp. 79-91,
Non-Patent Document 5: Chem. Lett., 2011, 40, 341-344
Non-Patent Document 6: J. Am. Chem. Soc., 2000, 122, 9870-9871
Non-Patent Document 7: J. Org. Chem., 2001, 66, 8177-8186
Non-Patent Document 8: Gazz. Chim. Ital., 1976, 106, 1131-1134
Non-Patent Document 9: Pure Appl. Chem., 1985, 57, 1845-1854
Non-Patent Document 10: Helv. Chim. Acta, 2001, 84, 230-242
Non-Patent Document 11: Tetrahedron Lett., 2006, 47, 5021-5024
Non-Patent Document 12: Angew. Chem. Int. Ed., 2009, 48, 5143-5147
Non-Patent Document 13: Chem. Commun., 2010, 46, 445-447
Non-Patent Document 14: Chem. Eur. J., 2010, 16, 12736-12745
Non-Patent Document 15: Angew. Chem. Int. Ed., 2011, 50, 2354-2358
Non-Patent Document 16: Synthesis, 2008, 2547-2550
Non-Patent Document 17: Chem. Eur. J., 2011, 17, 11143-11145
Non-Patent Document 18: Angew. Chem. Int. Ed., 2013, 52, 7500-7504

### Summary of Invention

### Technical Problem

However, natural menthol is greatly susceptible to weather and is therefore problematic in stably supply thereof. Regarding Patent Documents 1 and 2, in optical resolution of a racemic menthol, the content of 1-menthol is small, and the step of separating 1-menthol from the remaining 7 isomers other than 1-menthol is complicated. Regarding Non-Patent Document 1, the production method for 1-menthol from myrcene, as the starting material, requires a long process and the use of an expensive homogeneous rhodium complex as the catalyst for isomerization of diethylgeranylamine into the corresponding optically active enamine. The asymmetric synthesis method in Patent Document 3 has some risks in that piperitenone as the starting material is hardly available, that the method requires an expensive homogeneous complex such as a rhodium complex or a ruthenium complex and that the pressure in the hydrogenation reaction is high.

In the method for producing an optically active menthol described in Patent Document 4, in fact, geraniol and nerol must be separated through precision distillation, and for obtaining d-citronellol ((R)-citronellol) that is a starting material necessary for production of 1-menthol, expensive homogeneous catalysts for asymmetric hydrogenation of geraniol and nerol must be prepared separately, and therefore the number of the steps increases.

In the method for producing an optically active menthol described in Patent Document 5, high-purity neral or geranial must be taken out from geranial, neral or a mixture of geranial and neral, through precision distillation thereof. Subsequently, for obtaining d-citronellal ((R)-citronellal) that is a starting material necessary for production of 1-menthol, expensive rhodium catalysts differing in the chirality for asymmetric hydrogenation of neral and geranial must be prepared separately, and therefore the number of the steps increases. Further, the method has another problem in that the asymmetric hydrogenation must be carried out at a high hydrogen pressure.

The method for producing an optically active menthol described in Patent Document 6 uses citral as the starting material, and in the asymmetric hydrogenation step a) therein, hydrogen is used (Patent Document 6).

As described above, all the methods have some problems to be solved for efficient, economical and safe production, and a more simplified and efficient production method for an optically active menthol is desired.

An object of the present invention is to provide a method for producing an optically active isopulegol and an optically active menthol in short production steps, in which all the steps are catalytic reaction steps and therefore release of environment-polluting wastes can be minimized to the utmost limit, the energy efficiency is high, and the production expenses can be reduced.

### Solution to Problem

The present inventors have assiduously investigated for the purpose of solving the above-mentioned problems and, as a result, they have found that a) asymmetric isomerization of geraniol and/or nerol using an asymmetric metal catalyst gives a corresponding optically active citronellal having an extremely high optical purity, and they have further found a method that includes b) cyclizing the resultant optically active citronellal using a specific aluminum catalyst, thereby giving an n-selective, optically active isopulegol having an extremely high purity, or further subjecting the optically active isopulegol to cryogenic crystallization, thereby giving a high-purity optically active isopulegol, and c) hydrogenating these, thereby giving an optically active menthol, that is, a method for producing an optically active menthol from geraniol and/or nerol in short processes, and thus, the present invention has been completed. Specifically, the present invention includes the following aspects of inventions.
[1] A method for producing an optically active isopulegol represented by the following general formula (3), the method comprising a step of asymmetrically isomerizing a compound represented by the following general formula (1) in the presence of a ruthenium catalyst and a base, thereby giving an optically active citronellal represented by the following general formula (2) and a step of selectively cyclizing the optically active citronellal represented by the general formula (2) in the presence of an aluminum catalyst,
   wherein the aluminum catalyst is one obtained by reacting a hydroxy compound represented by the following general formula (5) or a hydroxy compound represented by the following general formula (6) and at least one aluminum compound selected from an alkylaluminum compound represented by the following general formula (7), a hydride aluminum compound represented by the following general formula (8), a linear aluminoxane represented by the following general formula (9) and a cyclic aluminoxane represented by the following general formula (10): 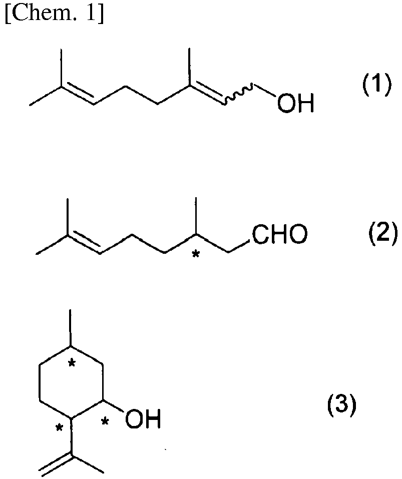 in the formula (1), the wavy line represents an (E) form and/or (Z) form of the double bond,
   in the formulae (2) and (3), * indicates an asymmetric carbon atom, 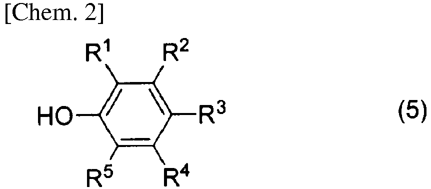 in the formula (5), R¹ and R⁵ each independently represents an aryl group having from 6 to 15 carbon atoms, which may have a substituent, a heteroaryl group having from 4 to 15 carbon atoms, which may have a substituent, or a cyclic alkyl group having from 3 to 15 carbon atoms, which may have a substituent; R², R³ and R⁴ each independently represents a hydrogen atom, an alkyl group having from 1 to 8 carbon atoms, an alkoxy group having from 1 to 8 carbon atoms, an aryl group having from 6 to 15 carbon atoms, which may have a substituent, a perfluoroalkyl group having from 1 to 4 carbon atoms, an aralkyl group having from 7 to 12 carbon atoms, a halogen atom, an organosilyl group, a dialkylamino group having from 1 to 4 carbon atoms, a thioalkoxy group having from 1 to 4 carbon atoms, a nitro group, or a polymer chain, 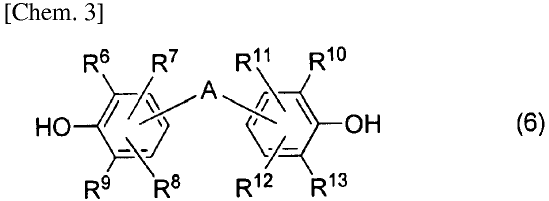 in the formula (6), R⁶, R⁹, R¹⁰ and R¹³ each independently represents an aryl group having from 6 to 15 carbon atoms, which may have a substituent, a heteroaryl group having from 4 to 15 carbon atoms, which may have a substituent, or a cyclic alkyl group having from 3 to 15 carbon atoms, which may have a substituent; R⁷, R⁸, R¹¹ and R¹² each independently represents a hydrogen atom, an alkyl group having from 1 to 8 carbon atoms, an alkoxy group having from 1 to 8 carbon atoms, an aryl group having from 6 to 15 carbon atoms, which may have a substituent, a perfluoroalkyl group having from 1 to 4 carbon atoms, an aralkyl group having from 7 to 12 carbon atoms, a halogen atom, an organosilyl group, a dialkylamino group having from 1 to 4 carbon atoms, a thioalkoxy group having from 1 to 4 carbon atoms, a nitro group, or a polymer chain,
   A represents (i) a single bond, (ii) a linear, branched or cyclic alkylene group having from 1 to 25 carbon atoms, which may have one or more of a substituent and an unsaturated bond, (iii) an arylene group having from 6 to 15 carbon atoms, which may have a substituent, (iv) a heteroarylene group having from 2 to 15 carbon atoms, which may have a substituent, or (v) a functional group or a hetero element, which is selected from the group consisting of -O-, -S-, -N(R¹⁴)-, -S(O)-, -C(O)-, -S(O)₂-, -P(R¹⁴)-, -(R¹⁴)P(O)- and -Si(R¹⁵R¹⁶)-; R¹⁴ to R¹⁶ each independently represents an alkyl group having from 1 to 6 carbon atoms, a cyclic alkyl group having from 5 to 8 carbon atoms, an aralkyl group having from 7 to 12 carbon atoms, which may have a substituent, or an aryl group having from 6 to 10 carbon atoms, which may have a substituent,

   AlHₖ(Lg)₃₋ₖ (7)

   in the formula (7), Al represents aluminum, Lg represents a branched or linear alkyl group having from 1 to 8 carbon atoms, a cyclic alkyl group having from 5 to 8 carbon atoms, or an aralkyl group having from 7 to 12 carbon atoms, which may have a substituent; and k indicates an integer of from 0 to 3,

   MAlH₄ (8)

   in the formula (8), Al represents aluminum, M represents lithium, sodium or potassium, 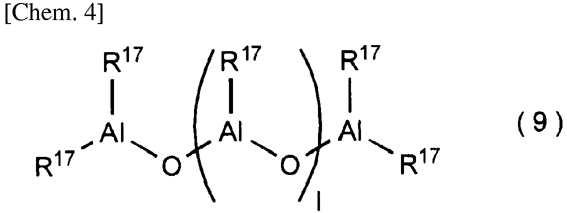 in the formula (9), Al represents aluminum, R¹⁷ represents a branched or linear alkyl group having from 1 to 8 carbon atoms, a cyclic alkyl group having from 5 to 8 carbon atoms, or an aralkyl group having from 7 to 12 carbon atoms, which may have a substituent, and a plurality of R¹⁷ may be the same or different; and 1 indicates an integer of from 0 to 40, 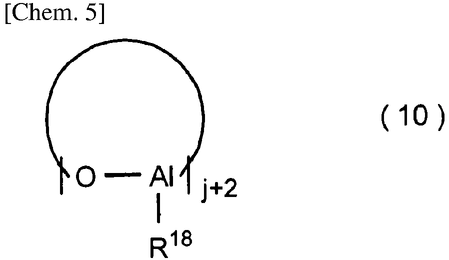 in the formula (10), Al represents aluminum, R¹⁸ represents a branched or linear alkyl group having from 1 to 8 carbon atoms, a cyclic alkyl group having from 5 to 8 carbon atoms, or an aralkyl group having from 7 to 12 carbon atoms, which may have a substituent; and j indicates an integer of from 0 to 40.
[2] The method for producing an optically active isopulegol according to [1], wherein the hydroxy compound represented by the general formula (5) is a hydroxy compound represented by the following general formula (5-a): 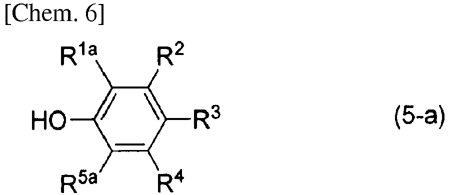 in the formula (5-a), R^{1a} represents an aryl group having from 6 to 15 carbon atoms, which may have a substituent, or a heteroaryl group having from 4 to 15 carbon atoms, which may have a substituent; R^{5a} represents a cyclic alkyl group having from 5 to 15 carbon atoms, which may have a substituent; R², R³ and R⁴ each independently represents a hydrogen atom, an alkyl group having from 1 to 8 carbon atoms, an alkoxy group having from 1 to 8 carbon atoms, an aryl group having from 6 to 15 carbon atoms, which may have a substituent, a perfluoroalkyl group having from 1 to 4 carbon atoms, an aralkyl group having from 7 to 12 carbon atoms, a halogen atom, an organosilyl group, a dialkylamino group having from 1 to 4 carbon atoms, a thioalkoxy group having from 1 to 4 carbon atoms, a nitro group, or a polymer chain.
[3] The method for producing an optically active isopulegol according to [1], wherein the hydroxy compound represented by the general formula (6) is a hydroxy compound represented by the following general formula (6-a): 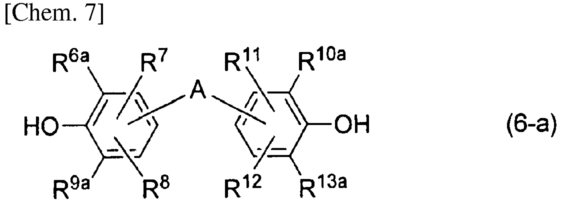 in the formula (6-a), R^{6a} and R^{10a} each independently represents an aryl group having from 6 to 15 carbon atoms, which may have a substituent, or a heteroaryl group having from 4 to 15 carbon atoms, which may have a substituent; R^{9a} and R^{13a} each represents a cyclic alkyl group having from 5 to 15 carbon atoms, which may have a substituent; R⁷, R⁸, R¹¹ and R¹² each independently represents a hydrogen atom, an alkyl group having from 1 to 8 carbon atoms, an alkoxy group having from 1 to 8 carbon atoms, an aryl group having from 6 to 15 carbon atoms, which may have a substituent, a perfluoroalkyl group having from 1 to 4 carbon atoms, an aralkyl group having from 7 to 12 carbon atoms, a halogen atom, an organosilyl group, a dialkylamino group having from 1 to 4 carbon atoms, a thioalkoxy group having from 1 to 4 carbon atoms, a nitro group, or a polymer chain,
   A represents (i) a single bond, (ii) a linear, branched or cyclic alkylene group having from 1 to 25 carbon atoms, which may have one or more of a substituent and an unsaturated bond, (iii) an arylene group having from 6 to 15 carbon atoms, which may have a substituent, (iv) a heteroarylene group having from 2 to 15 carbon atoms, which may have a substituent, or (v) a functional group or a hetero element, which is selected from the group consisting of -O-, -S-, -N(R¹⁴)-, -S(O)-, -C(O)-, -S(O)₂-, -P(R¹⁴)-, -(R¹⁴)P(O)- and -Si(R¹⁵R¹⁶)-; R¹⁴ to R¹⁶ each independently represents an alkyl group having from 1 to 6 carbon atoms, a cyclic alkyl group having from 5 to 8 carbon atoms, an aralkyl group having from 7 to 12 carbon atoms, which may have a substituent, or an aryl group having from 6 to 10 carbon atoms, which may have a substituent.
[4] The method for producing an optically active isopulegol according to any one of [1] to [3], wherein the cyclization reaction is carried out in the presence of at least one compound of the following compounds I and II:
   I. at least one acid,
   II. at least one compound selected from the group containing an aldehyde except citronellal, an acid anhydride, a ketone, an acid halide, an epoxy compound and a vinyl ether.
[5] The method for producing an optically active isopulegol according to any one of [1] to [4], wherein the ruthenium catalyst is a ruthenium compound represented by the following general formula (11):

   [RuₘLₙWₚU_{q}]ᵣZₛ (11)

   in the formula, L represents an optically active phosphine ligand; W represents a hydrogen atom, a halogen atom, an acyloxy group, an aryl group, a diene or an anion; U represents a hydrogen atom, a halogen atom, an acyloxy group, an aryl group, a diene, an anion or a ligand except L; Z represents an anion, an amine or an optically active nitrogen-containing compound; m, n and r each independently indicate an integer of from 1 to 5; p, q and s each independently indicate an integer of from 0 to 5; and p + q + s is 1 or more.
[6] The method for producing an optically active isopulegol according to any one of [1] to [5], wherein the base is a salt of an alkali metal or alkaline earth metal, or a quaternary ammonium salt.
[7] A method for producing an optically active menthol, comprising a step of preparing an optically active isopulegol according to the method as described in any one of [1] to [6], and a step of hydrogenating the optically active isopulegol prepared.
[8] A method for producing an optically active menthol, comprising the following steps:
   A-1) preparing an optically active citronellal through asymmetric isomerization of geraniol or nerol,
   B-1) preparing an optically active isopulegol through cyclization reaction of the optically active citronellal with an acidic catalyst, and
   C-1) hydrogenating the optically active isopulegol, thereby giving the optically active menthol.
[9] A method for producing an optically active menthol, comprising the following steps:
   A-2) preparing an optically active citronellal through asymmetric isomerization of geraniol or nerol,
   B-2) preparing an optically active isopulegol through cyclization reaction of the optically active citronellal with an acidic catalyst,
   D-2) preparing an isopulegol having a further higher purity through cryogenic recrystallization of the optically active isopulegol, and
   E-2) hydrogenating the optically active isopulegol prepared in the step D-2, thereby giving the optically active menthol.
[10] A method for producing an optically active menthol, comprising the following steps:
   A-3) preparing d-citronellal through asymmetric isomerization of geraniol or nerol,
   B-3) preparing 1-isopulegol through cyclization reaction of the d-citronellal with an acidic catalyst, and
   C-3) hydrogenating the 1-isopulegol, thereby giving 1-menthol.
[11] A method for producing an optically active menthol, comprising the following steps:
   A-4) preparing d-citronellal through asymmetric isomerization of geraniol or nerol,
   B-4) preparing 1-isopulegol through cyclization reaction of the d-citronellal with an acidic catalyst,
   D-4) preparing 1-isopulegol having a further higher purity through cryogenic recrystallization of the 1-isopulegol, and
   E-4) hydrogenating the 1-isopulegol prepared in the step D-4, thereby giving 1-menthol.

### Advantageous Effects of Invention

According to the production method of the present invention, geraniol and/or nerol are/is asymmetrically isomerized in the step of the first stage, thereby giving an optically active citronellal, and a specific ruthenium catalyst is used, thereby giving an optically active citronellal having a high optical purity.

The asymmetric isomerization in the production method of the present invention does not require the step in which geraniol and/or nerol is asymmetrically hydrogenated, followed by post-oxidation of the resultant citronellal, like in the previously-disclosed report (Patent Document 4), and therefore the steps can be shortened.

In the step of the second stage, the optically active citronellal obtained in the step of the first stage is cyclized with an aluminum catalyst, and therefore, an optically active isopulegol can be produced highly selectively between four types of isomers.

In the optional step of the third stage, the optically active isopulegol obtained in the step of the second stage is subjected to cryogenic crystallization at a low temperature, and an optically active isopulegol having further higher chemical purity and optical purity can be thereby produced.

In the step of the fourth stage, the optically active isopulegol obtained in the step of the second stage or the optically active isopulegol obtained in the third stage is hydrogenated with a hydrogenation catalyst, thereby giving an optically active menthol.

As a result, as the production method for an optically active menthol through chemical synthesis, the optically active menthol can be produced in a shortest process from starting materials. In addition, citronellal obtained through asymmetric isomerization of geraniol or nerol has extremely high chemical purity and optical purity, and by cyclizing the citronellal with a highly-selective cyclization catalyst, 1-isopulegol having a high purity can be produced without requiring any crystallization operation. Further, all the production steps expect the step of cryogenic crystallization are steps using a catalyst, and therefore the production method of the present invention releases few environment-polluting wastes and the production expenses can be reduced.

### Brief Description of Drawings

[FIG. 1] FIG. 1 is a view showing a ¹N-NMR spectrum of the solid obtained by reacting 2-cyclohexyl-6-phenylphenol and triethylaluminium in Example 5.
[FIG. 2] FIG. 2 is an enlarged view on the low magnetic field side of the ¹N-NMR spectrum shown in FIG. 1.
[FIG. 3] FIG. 3 is a view showing a ¹N-NMR spectrum of 2-cyclohexyl-6-phenylphenol (CPP).
[FIG. 4] FIG. 4 is an enlarged view on the low magnetic field side of ¹N-NMR spectrum shown in FIG. 3.

### Description of Embodiments

The present invention is described in detail hereinunder.

In this description, "% by weight" and "part by weight" are the same as "% by mass" and "part by mass", respectively.

The production method for an optically active menthol in the present invention is carried out according to the method shown in Scheme 1. In this description, * in the chemical structures indicates an asymmetric carbon.

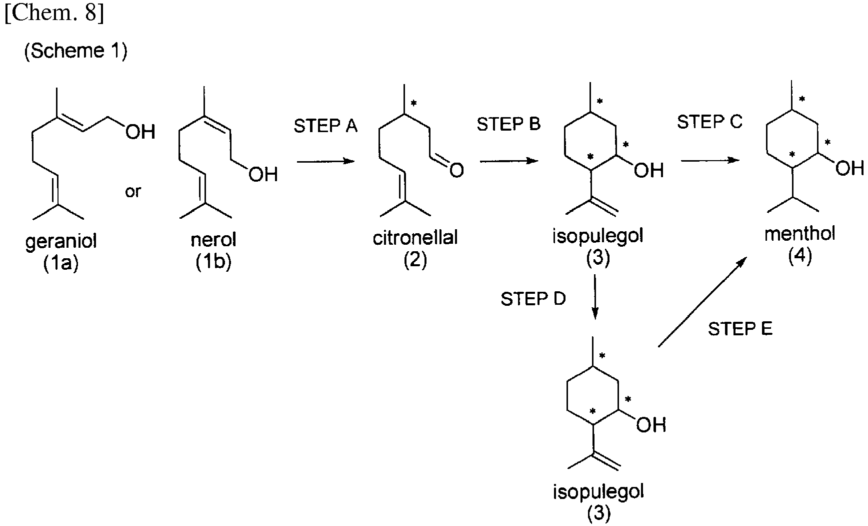

### <Step A>

The step A shown in the scheme 1 includes asymmetrically isomerizing at least one of geraniol and nerol using an asymmetric isomerization catalyst, thereby producing an optically active citronellal.

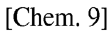

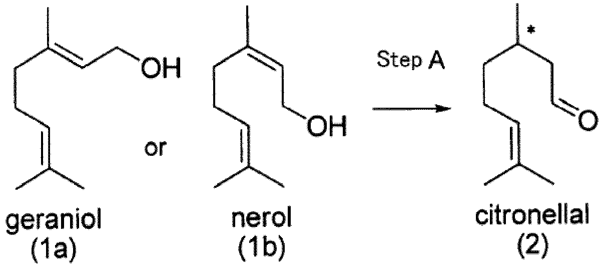

### <Step A: Asymmetric isomerization catalyst>

### (Ruthenium catalyst)

### Step A in Scheme 1:

The asymmetric isomerization catalyst is described. As the ruthenium catalyst that is the asymmetric isomerization catalyst, preferable examples thereof include a complex including ruthenium and a ligand. Preferably, the ligand is an asymmetric ligand, in which, however, constituent component other than the ligand may be an optically active form.

The asymmetric ligand for use in producing the ruthenium complex which is the asymmetric isomerization catalyst, may be any and every optically active compound having an optically active site and capable of being used as an asymmetric ligand. Examples of the asymmetric ligand include, for example, those described in Catalytic Asymmetric Synthesis (Wiley-VCH, 2000), Handbook of Enantioselective Catalysis with Transition Metal Complex (VCH, 1993), ASYMMETRIC CATALYSIS IN ORGANIC SYNTHESIS (John Wiley & Sons Inc. (1994)), WO 2005/070875, etc.

This is described more concretely. Examples of the asymmetric ligand for use in the present invention include, for example, a monodentate ligand, a bidentate ligand, a tridentate ligand, a quadridentate ligand, etc. For example, examples thereof include optically active phosphine compounds, optically active amine compounds, optically active alcohol compounds, optically active sulfur compounds, optically active carbene compounds, etc. Preferable examples thereof include optically active phosphine compounds.

As the optically active phosphine compounds, examples thereof include optically active bidentate phosphine ligands represented by the following general formula (12):

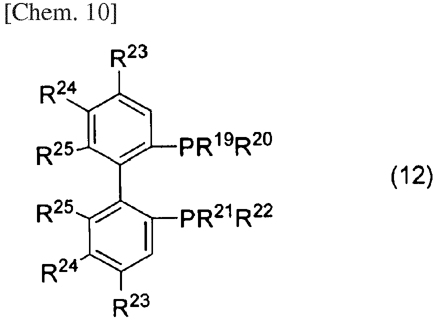

In the formula (12), R¹⁹ to R²² each independently represents an aromatic group which may have a substituent or a cycloalkyl group having from 3 to 10 carbon atoms or R¹⁹ and R²⁰, and R²¹ and R²² each may form a hetero ring with the adjacent phosphorus atom; R²³ and R²⁴ each independently represents hydrogen atom, an alkyl group having from 1 to 5 carbon atoms, an alkoxy group having from 1 to 5 carbon atoms, a di(C₁₋₅ alkyl)amino group, a 5- to 8-membered cyclic amino group or a halogen atom; R²⁵ represents an alkyl group having from 1 to 5 carbon atoms, an alkoxy group having from 1 to 5 carbon atoms, a di(C₁₋₅ alkyl)amino group, a 5- to 8-membered cyclic amino group or a halogen atom; or R²³ and R²⁴, and R²⁴ and R²⁵ each may bond to each other to form a condensed benzene ring, a condensed substituted benzene ring, trimethylene group, tetramethylene group, pentamethylene group, methylenedioxy group, ethylenedioxy group or trimethylenedioxy group.

In the general formula (12), R¹⁹ to R²² each independently represents an aromatic group which may have a substituent or a cyclic alkyl group having from 3 to 10 carbon atoms, or R¹⁹ and R²⁰, and R²¹ and R²² each may form a hetero ring with the adjacent phosphorus atom.

In the aromatic group which may have a substituent, examples of the aromatic group include a hydrocarbon aromatic group such as phenyl group, naphthyl group and phenanthryl group; and a heterocyclic aromatic group such as pyrrolyl group, pyridyl group, pyrazyl group, quinolyl group, isoquinolyl group and imidazolyl group.

Here, specific examples of the substituent include, for example, an alkyl group having from 1 to 12 carbon atoms such as methyl group, ethyl group, n-propyl group, isopropyl group, n-butyl group, isobutyl group, sec-butyl group, tert-butyl group, n-pentyl group, isopentyl group, neopentyl group, hexyl group, heptyl group, octyl group, nonyl group, decyl group, undecyl group and dodecyl group; a lower alkoxy group having from 1 to 4 carbon atoms such as methoxy group, ethoxy group, n-propoxy group, isopropoxy group, n-butoxy group, isobutoxy group, sec-butoxy group and tert-butoxy group; an aryl group such as phenyl group, α-naphthyl group, β-naphthyl group and phenanthryl group; an aralkyl group having from 7 to 13 carbon atoms such as benzyl group, α-phenylethyl group, β-phenylethyl group, α-phenylpropyl group, β-phenylpropyl group, γ-phenylpropyl group and naphthylmethyl group; a tri-substituted organosilyl group, for example, a tri-C₁₋₆ alkylsilyl group such as trimethylsilyl group, triethylsilyl group, triisopropylsilyl group, dimethylisopropylsilyl group, diethylisopropylsilyl group, dimethyl(2,3-dimethyl-2-butyl)silyl group, tert-butyldimethylsilyl group and dimethylhexylsilyl group, a di-C₁₋₆ alkyl-C₆₋₁₈ arylsilyl group such as dimethylcumylsilyl group, a di-C₆₋₁₈ aryl-C₁₋₆ alkylsilyl group such as tert-butyldiphenylsilyl group and diphenylmethylsilyl group, a tri-C₆₋₁₈ arylsilyl group such as triphenylsilyl group, a tri-C₇₋₁₉ aralkylsilyl group such as tribenzylsilyl group and a tri-p-xylylsilyl group; a halogen atom such as fluorine, chlorine, bromine and iodine; a nitro group, etc.

Specific examples of the cycloalkyl group having from 3 to 10 carbon atoms, which may have a substituent, include cyclopentyl group, cyclobutyl group, cyclopentyl group, cyclohexyl group, cycloheptyl group, cyclooctyl group, cyclononyl group, cyclodecyl group and octahydronaphthyl group.

Here, specific examples of the substituent include, for example, an alkyl group having from 1 to 12 carbon atoms such as methyl group, ethyl group, n-propyl group, isopropyl group, n-butyl group, isobutyl group, sec-butyl group, tert-butyl group, n-pentyl group, isopentyl group, neopentyl group, hexyl group, heptyl group, octyl group, nonyl group, decyl group, undecyl group and dodecyl group; a lower alkoxy group having from 1 to 4 carbon atoms such as methoxy group, ethoxy group, n-propoxy group, isopropoxy group, n-butoxy group, isobutoxy group, sec-butoxy group and tert-butoxy group; an aryl group such as phenyl group, α-naphthyl group, β-naphthyl group and phenanthryl group; an aralkyl group having from 7 to 13 carbon atoms such as benzyl group, α-phenylethyl group, β-phenylethyl group, α-phenylpropyl group, β-phenylpropyl group, γ-phenylpropyl group and naphthylmethyl group; a tri-substituted organosilyl group, for example, a tri-C₁₋₆ alkylsilyl group such as trimethylsilyl group, triethylsilyl group, triisopropylsilyl group, dimethylisopropylsilyl group, diethylisopropylsilyl group, dimethyl(2,3-dimethyl-2-butyl)silyl group, tert-butyldimethylsilyl group and dimethylhexylsilyl group, a di-C₁₋₆ alkyl-C₆₋₁₈ arylsilyl group such as dimethylcumylsilyl group, a di-C₆₋₁₈ aryl-C₁₋₆ alkylsilyl group such as tert-butyldiphenylsilyl group and diphenylmethylsilyl group, a tri-C₆₋₁₈ arylsilyl group such as triphenylsilyl group, a tri-C₇₋₁₉ aralkylsilyl group such as tribenzylsilyl group and a tri-p-xylylsilyl group; a halogen atom such as fluorine, chlorine, bromine and iodine; a nitro group, etc.

Specific examples of the hetero ring to be formed by R¹⁹ and R²⁰ or R²¹ and R²² each bonding to the adjacent phosphorus atom include phosphole, tetrahydrophosphole and phosphorinane. The hetero ring may have, as substituents, from 1 to 4 functional groups inert to the reaction in the present invention. Examples of the substituent include, for example, an alkyl group having from 1 to 4 carbon atoms, an alkoxy group having from 1 to 4 carbon atoms and a halogen atom.

In the general formula (12), R²³ and R²⁴ each independently represents hydrogen atom, an alkyl group having from 1 to 5 carbon atoms, an alkoxy group having from 1 to 5 carbon atoms, a di(C₁₋₅ alkyl)amino group, a 5- to 8-membered cyclic amino group or a halogen atom.

Specific examples of the alkyl group having from 1 to 5 carbon atoms, which is represented by R²³ and R²⁴, include, for example, methyl group, ethyl group, n-propyl group, isopropyl group, n-butyl group, isobutyl group, sec-butyl group, tert-butyl group and a pentyl group.

Specific examples of the alkoxy group having from 1 to 5 carbon atoms, which is represented by R²³ and R²⁴, include, for example, methoxy group, ethoxy group, n-propoxy group, isopropoxy group, n-butoxy group, isobutoxy group, sec-butoxy group, tert-butoxy group and pentoxy group.

Specific examples of the di(C₁₋₅ alky)amino group which is represented by R²³ and R²⁴ include dimethylamino group, diethylamino group, di-n-propylamino group, diisopropylamino group, di-n-butylamino group, di-isobutylamino group, di-sec-butylamino group, di-tert-butylamino group and dipentylamino group.

Specific examples of the 5- to 8-membered cyclic amino group which is represented by R²³ and R²⁴ include pyrrolidino group and piperidino group.

Specific examples of the halogen atom which is represented by R²³ and R²⁴ include fluorine atom, chlorine atom, bromine atom and iodine atom.

Of those, examples of preferred R²³ and R²⁴ include hydrogen atom; an alkyl group having from 1 to 4 carbon atoms such as methyl group, ethyl group, propyl group, isopropyl group, n-butyl group, tert-butyl group and trifluoromethyl group; an alkoxy group such as methoxy group, ethoxy group, n-propoxy group, isopropoxy group, n-butoxy group and tert-butoxy group; a dialkylamino group such as dimethylamino group and diethylamino group; a 5- to 8-membered cyclic amino group such as pyrrolidino group and piperidino group, etc.

Examples of more preferred R²³ and R²⁴ include hydrogen atom and methoxy group.

In the general formula (12), R²⁵ represents an alkyl group having from 1 to 5 carbon atoms, an alkoxy group having from 1 to 5 carbon atoms, a di(C₁₋₅ alkyl)amino group, a 5- to 8-membered cyclic amino group or a halogen atom.

Specific examples of the alkyl group having from 1 to 5 carbon atoms, which is represented by R²⁵, include, for example, methyl group, ethyl group, n-propyl group, isopropyl group, n-butyl group, isobutyl group, sec-butyl group, tert-butyl group and a pentyl group.

Specific examples of the alkoxy group having from 1 to 5 carbon atoms, which is represented by R²⁵, include, for example, methoxy group, ethoxy group, n-propoxy group, isopropoxy group, n-butoxy group, isobutoxy group, sec-butoxy group, tert-butoxy group and pentoxy group.

Specific examples of the di(C₁₋₅ alky)amino group which is represented by R²⁵ include dimethylamino group, diethylamino group, di-n-propylamino group, diisopropylamino group, di-n-butylamino group, di-isobutylamino group, di-sec-butylamino group, di-tert-butylamino group and dipentylamino group.

Specific examples of the 5- to 8-membered cyclic amino group which is represented by R²⁵ include pyrrolidino group and piperidino group.

Specific examples of the halogen atom which is represented by R²⁵ include fluorine atom, chlorine atom, bromine atom and iodine atom.

Of those, examples of preferred R²⁵ include an alkyl group having from 1 to 4 carbon atoms such as methyl group, ethyl group, propyl group, isopropyl group, n-butyl group, tert-butyl group and trifluoromethyl group; an alkoxy group such as methoxy group, ethoxy group, n-propoxy group, isopropoxy group, n-butoxy group and tert-butoxy group; a dialkylamino group such as dimethylamino group and diethylamino group; a 5- to 8-membered cyclic amino group such as pyrrolidine group and piperidino group, etc.

Examples of more preferred R²³ and R²⁴ include methyl group and methoxy group.

In the general formula (12), R²³ and R²⁴, and R²⁴ and R²⁵ each may bond to each other to form a condensed benzene ring, a condensed substituted benzene ring, trimethylene group, tetramethylene group, pentamethylene group, methylenedioxy group, ethylenedioxy group or trimethylenedioxy group. Of those, preferred examples thereof include a condensed benzene ring, a condensed substituted benzene ring, trimethylene group, tetramethylene group, pentamethylene group, methylenedioxy group, ethylenedioxy group or trimethylenedioxy group that is formed by R²⁴ and R²⁵ bonding to each other. More preferred examples thereof include a condensed benzene ring, a condensed substituted benzene ring, tetramethylene group, methylenedioxy group, methylenedioxy group or ethylenedioxy group that is formed by R²⁴ and R²⁵ bonding to each other.

The condensed benzene ring, the condensed substituted benzene ring, the trimethylene group, the tetramethylene group, the pentamethylene group, the methylenedioxy group, the ethylenedioxy group or the trimethylenedioxy group may have, as the substituent, a functional group inert to asymmetric synthesis reaction, and preferably the number of the substituents is within a range of from 0 to 4. Examples of the substituent include, for example, an alkyl group having from 1 to 4 carbon atoms such as methyl group, ethyl group, n-propyl group, isopropyl group, n-butyl group, isobutyl group, sec-butyl group and tert-butyl group; hydroxyl group; an alkoxy group having rom 1 to 4 carbon atoms such as methoxy group, ethoxy group, n-propoxy group, isopropoxy group, n-butoxy group, isobutoxy group, sec-butoxy group and tert-butoxy group; a halogen atom such as fluorine, chlorine, bromine, iodine, etc.

Examples of the optically active bidentate phosphine ligand preferably used in the general formula (12) include, for example, a tertiary phosphine described in JP-A 61-63690 and JP-A 62-265293, and concrete examples thereof include 2,2'-bis(diphenylphosphino)-1,1'-binaphthyl (BINAP), 2,2'-bis(di(p-tolylphosphino)-1,1'-binaphthyl [p-Tol-BINAP], 2,2'-bis(di(3,5-xylyl)phosphino)-1,1'-binaphthyl(DM-BINAP), 2,2'-bis(di(3,5-di-tert-butylphenyl)phosphino)-1,1'-binaphthyl (T-Bu-2-BINAP), 2,2'-bis[di(4-methoxy-3,5-dimethylphenyl)phosphino]-1,1'-binaphthyl (DMM-BINAP), 2,2'-bis(dicyclohexylphosphino)-1,1'-binaphthyl (Cy-BINAP) and 2,2'-bis(dicyclopentylphosphino)-1,1'-binaphthyl (Cp-BINAP).

Further, examples of the optically active bidentate phosphine ligand preferably used in the general formula (12) include, for example, a tertiary phosphine described in JP-A 4-139140, and concrete examples thereof include: 2,2'-bis(diphenylphosphino)-5,5',6,6',7,7',8,8'-octahydrobinaphthyl (H₈-BINAP), 2,2'-bis(di-p-tolylphosphino)-5,5',6,6',7,7',8,8'-octahydrobinaphthyl (p-Tol-H₈-BINAP), 2,2'-bis(di-(3,5-xylyl)phosphino)-5,5',6,6',7,7',8,8'-octahydrobinaphthyl (DM-H₈-BINAP), and 2,2'-bis(di-(4-methoxy-3,5-dimehtylphenyl)phosphino)-5,5',6,6',7,7',8,8'-octahydrobinaphthyl (DMM-H₈-BINAP).

Still further, examples of the optically active bidentate phosphine ligand preferably used in the general formula (12) include, for example, a tertiary phosphine described in JP-A 11-269185, and concrete examples thereof include:
((5,6),(5',6')-bis(methylenedioxy)biphenyl-2,2'-diyl)bis(diphenylphosphine) (SEGPHOS),
((5,6),(5',6')-bis(methylenedioxy)biphenyl-2,2'-diyl)bis(di-p-tolylphosphine) (p-Tol-SEGPHOS),
((5,6),(5',6')-bis(methylenedioxy)biphenyl-2,2'-diyl)bis(di-3,5-xylylphosphine) (DM-SEGPHOS), ((5,6),(5',6')-bis(methylenedioxy)biphenyl-2,2'-diyl)bis(di-4-methoxy-3,5-dimehtylphenylphosphine) (DMM-SEGPHOS),
((5,6),(5',6')-bis(methylenedioxy)biphenyl-2,2'-diyljbis(di-4-methoxy-3,5-di-tert-butyl phenylphosphine) (DTBM-SEGPHOS),
((5,6),(5',6')-bis(methylenedioxy)biphenyl-2,2'-diyl)bis(dicyclohexylphosphine) (Cy-SEGPHOS).

Other than the above-mentioned optically active bidentate phosphine ligands, the following optically active bidentate phosphine ligands are mentioned as those corresponding to the general formula (12):
2,2'-dimethyl-6,6'-bis(diphenylphosphino)-1,1'-biphenyl (BIPHEMP),
2,2'-dimethyl-6,6'-bis(di-p-tolylphosphino)-1,1'-biphenyl (p-Tol-BIPHEMP),
2,2'-dimethyl-6,6'-bis(di-3,5-xylylphosphino)-1,1'-biphenyl (DM-BIPHEMP),
2,2'-dimethyl-6,6'-bis(di-4-methoxy-3,5-dimethylphenylphosphino)-1,1'-biphenyl (DMM-BIPHEMP),
2,2'-dimethyl-6,6'-bis(di-4-t-butoxy-3,5-dimethylphenylphosphino)-1,1'-biphenyl (DTBM-BIPHEMP),2,2'-dimethyl-6,6'-bis(dicyclohexylphosphino)-1,1'-biphenyl (Cy-BIPHEMP), 2,2'-dimethoxy-6,6'-bis(diphenylphosphino)-1,1'-biphenyl (MeO-BIPHEMP), 2,2'-dimethoxy-6,6'-bis(di-p-tolylphosphino)-1,1'-biphenyl (p-Tol-MeO-BIPHEMP), 2,2'-dimethoxy-6,6'-bis(di-3,5-xylylphosphino)-1,1'-biphenyl (DM-MeOBIPHEMP),
2,2'-dimethoxy-6,6'-bis(di-4-methoxy-3,5-dimethylphenylphosphino)-1,1'-biphenyl (DMM-MeO-BIPHEMP),
2,2'-dimethoxy-6,6'-bis(di-4-t-butoxy-3,5-dimethylphenylphosphino)-1,1'-biphenyl (DTBM-MeO-BIPHEMP),
2,2'-dimethoxy-6,6'-bis(dicyclohexylphosphino)-1,1'-biphenyl (Cy-MeO-BIPHEMP),
2,2'-dimethyl-3,3'-dichloro-4,4'-dimethyl-6,6'-bis(di-p-tolylphosphino)-1,1'-biphenyl (p-Tol-CM-BIPHEMP),
2,2'-dimethyl-3,3'-dichloro-4,4'-dimethyl-6,6'-bis(di-3,5-xylylphosphino)-1,1'-biphenyl (DM-CM-BIPHEMP), 2,2'-dimethyl-3,3'-dichloro-4,4'-dimethyl-6,6'-bis(di-4-methoxy-3,5-dimethylphenylphosphino)-1,1'-biphenyl (DMM-CM-BIPHEMP).

In the present invention, a ruthenium complex containing the above-mentioned ligand and ruthenium is used for asymmetric isomerization reaction. As the optically active ruthenium complex for the asymmetric isomerization reaction, for example, preferred examples thereof include the compounds represented by the following general formula (11):

[RuₘLₙWₚU_{q}]ᵣZₛ (11)

In the formula, L represents an optically active phosphine ligand; W represents hydrogen atom, a halogen atom, an acyloxy group, an aryl group, a diene or an anion; U represents hydrogen atom, a halogen atom, an acyloxy group, an aryl group, a diene, an anion or a ligand other than L; Z represents an anion, amine or an optically active nitrogen-containing compound; m, n and r each independently indicates an integer of from 1 to 5; p, q and s each independently indicates an integer of from 0 to 5, and p + q + s is 1 or more.

In the general formula (11), examples of the ligand represented by L include the optically active bidentate phosphine ligand represented by the above-mentioned general formula (12).

In the general formula (11), W represents hydrogen atom, a halogen atom, an acyloxy group, an aryl group, a diene or an anion.

Examples of the halogen atom represented by W in the general formula (11) include, for example, fluorine atom, chlorine atom, bromine atom and iodine atom.

Examples of the acyloxy group represented by W in the general formula (11) include, for example, formyloxy group, acetoxy group, propionyloxy group, butyloxy group, benzoyloxy group, etc.

Examples of the aryl group represented by W in the general formula (11) include, for example, aromatic monocyclic or polycyclic groups, such as phenyl group, naphthyl group, anthranyl group, phenanthryl group, indenyl group, mesityl group, dibenzyl group, etc.

Examples of the diene represented by W in the general formula (11) include, for example, butadiene, cyclooctadiene (cod), norbornadiene (nod), etc.

Examples of the anion represented by W in the general formula (11) include, for example, nitrate ion, nitrite ion, sulfate ion, sulfite ion, sulfonate ion (methanesulfonate ion, benzenesulfonate ion, p-toluenesulfonate ion, camphorsulfonate ion, trifluoromethanesulfonate ion, etc.), sulfamate ion, carbonate ion, hydroxide ion, carboxylate ion (formate ion, acetate ion, propionate ion, gluconate ion, oleate ion, oxalate ion, benzoate ion, phthalate ion, trifluoroacetate ion, etc.), sulfide ion, thiocyanate ion, phosphate ion, pyrophosphate ion, oxide ion, phosphide ion, chlorate ion, perchlorate ion, iodate ion, hexafluorosilicate ion, cyanide ion, borate ion, metaborate ion, borofluoride ion, etc.

In the general formula (11), U represents hydrogen atom, a halogen atom, an acyloxy group, an aryl group, a diene, an anion or a ligand other than L.

Examples of the halogen atom represented by U in the general formula (11) include, for example, fluorine atom, chlorine atom, bromine atom and iodine atom.

Examples of the acyloxy group represented by U in the general formula (11) include, for example, formyloxy group, acetoxy group, propionyloxy group, butyloxy group, benzoyloxy group, etc.

Examples of the aryl group represented by U in the general formula (11) include, for example, aromatic monocyclic or polycyclic groups, such as phenyl group, naphthyl group, anthranyl group, phenanthryl group, indenyl group, mesityl group, dibenzyl group, etc.

Examples of the diene represented by U in the general formula (11) include, for example, butadiene, cyclooctadiene (cod), norbornadiene (nod), etc.

Examples of the anion represented by U in the general formula (11) include, for example, nitrate ion, nitrite ion, sulfate ion, sulfite ion, sulfonate ion (methanesulfonate ion, benzenesulfonate ion, p-toluenesulfonate ion, camphorsulfonate ion, trifluoromethanesulfonate ion, etc.), sulfamate ion, carbonate ion, hydroxide ion, carboxylate ion (formate ion, acetate ion, propionate ion, gluconate ion, oleate ion, oxalate ion, benzoate ion, phthalate ion, trifluoroacetate ion, etc.), sulfide ion, thiocyanate ion, phosphate ion, pyrophosphate ion, oxide ion, phosphide ion, chlorate ion, perchlorate ion, iodate ion, hexafluorosilicate ion, cyanide ion, borate ion, metaborate ion, borofluoride ion, etc.

Examples of the ligand except L, which is represented by U in the general formula (11), include, for example, N,N-dimethylformamide (DMF), acetone, chloroform, a nitrile (acetonitrile, benzonitrile, etc.), a cyanide (methylisocyanide, phenylisocyanide, etc.), an aromatic compound (benzene, p-cymene, 1,3,5-trimethylbenzene (mesitylene), hexamethylbenzene, etc.), an olefin (ethylene, propylene, cycloolefin, etc.), a phosphorus compound (phosphane compounds such as triphenyl phosphine, tritolyl phosphine, trimethyl phosphine, triethyl phosphine, methyldiphenyl phosphine, dimethylphenyl phosphine, diphenylphosphinomethane (dppm), diphenylphosphinoethane (dppe), diphenylphosphinopropane (dppp), diphenylphosphinobutane (dppb) and diphenylphosphinoferrocene (dppf), phosphite compounds such as trimethyl phosphite, triethyl phosphite and triphenyl phosphite), an amine compound (ammonia; an aliphatic amine such as methylamine, ethylamine, n-propylamine, isopropylamine, n-butylamine, s-butylamine, tert-butylamine and cyclohexylamine; an aromatic amine such as aniline and dimethylaniline; a nitrogen-containing aromatic heterocyclic compound such as pyridine (py) and dimethylaminopyridine; a nitrogen-containing aliphatic heterocyclic compound such as pyrrolidine and piperazine; a diamine such as ethylenediamine (en), propylenediamine, triethylenediamine, tetramethylethylenediamine (TMEDA), bipyridine (bpy) and phenanthroline (phen)), a sulfur compound (dimethyl sulfide, diethyl sulfide, dipropyl sulfide, dibutyl sulfide, etc.), etc.

In the general formula (11), Z represents an anion, amine or an optically active nitrogen-containing compound.

Examples of the anion represented by Z in the general formula (11) include, for example, nitrate ion, nitrite ion, sulfate ion, sulfite ion, sulfonate ion (methanesulfonate ion, benzenesulfonate ion, p-toluenesulfonate ion, camphorsulfonate ion, trifluoromethanesulfonate ion, etc.), sulfamate ion, carbonate ion, hydroxide ion, carboxylate ion (formate ion, acetate ion, propionate ion, gluconate ion, oleate ion, oxalate ion, benzoate ion, phthalate ion, trifluoroacetate ion, etc.), sulfide ion, thiocyanate ion, phosphate ion, pyrophosphate ion, oxide ion, phosphide ion, chlorate ion, perchlorate ion, iodate ion, hexafluorosilicate ion, cyanide ion, borate ion, metaborate ion, borofluoride ion, etc.

Examples of the amine represented by Z in the general formula (11) include, for example, an aliphatic amine such as methylamine, ethylamine, n-propylamine, isopropylamine, n-butylamine, s-butylamine, tert-butylamine and cyclohexylamine; an aromatic amine such as aniline and dimethylaniline; a nitrogen-containing aromatic heterocyclic compound such as pyridine (py) and dimethylaminopyridine; a nitrogen-containing aliphatic heterocyclic compound such as pyrrolidine and piperazine; a diamine such as ethylenediamine (en), propylenediamine, triethylenediamine, tetramethylethylenediamine (TMEDA), bipyridine (bpy) and phenanthroline (phen); a sulfur compound (dimethyl sulfide, diethyl sulfide, dipropyl sulfide, dibutyl sulfide, etc.), etc.

Examples of the optically active nitrogen-containing compound represented by Z in the general formula (11) include an optically active diamine compound represented by the following general formula (13).

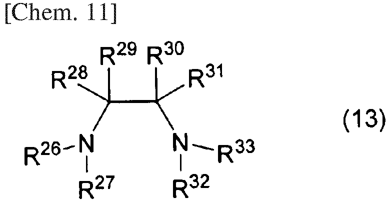

In the formula (13), R²⁶, R²⁷, R³² and R³³ each independently represents hydrogen atom, a saturated or unsaturated hydrocarbon group, an aryl group, an alaryl group, an urethane group, a sulfonyl group, etc.; R²⁸, R²⁹, R³⁰ and R³¹ each independently represents hydrogen atom, an alkyl group, an aromatic monocyclic or polycyclic group, a saturated or unsaturated hydrocarbon group, a cyclic alkyl group, etc.; and the carbon atom to which R²⁸, R²⁹, R³⁰ and R³¹ bond is an asymmetric center.

Examples of the optically active diamine compound represented by the general formula (13) include, for example, 1,2-diphenylethylenediamine, 1,2-cyclohexanediamine, 1,2-cycloheptanediamine, 2,3-dimethylbutanediamine, 1-methyl-2,2-diphenylethylenediamine, 1-isobutyl-2,2-diphenylethylenediamine, 1-isopropyl-2,2-diphenylethylenediamine, 1-methyl-2,2-di(p-methoxyphenyl)ethylenediamine, 1-isobutyl-2,2-di(p-methoxyphenyl)ethylenediamine, 1-isopropyl-2,2-di(p-methoxyphenyl)ethylenediamine, 1-benzyl-2,2-di(p-methoxyphenyl)ethylenediamine, 1-methyl-2,2-dinaphthylethylenediamine, 1-isobutyl-2,2-dinaphthylethylenediamine, 1-isopropyl-2,2-dinaphthylethylenediamine, 2-methylamino-1-phenylethylamine, 2-ethylamino-1-phenylethylamine, 2-n-propylamino-1-phenylethylamine, 2-i-propylamino-1-phenylethylamine, 2-n-butylamino-1-phenylethylamine, 2-tert-butylamino-1-phenylethylamine, 2-cyclohexylamino-1-phenylethylamine, 2-benzylamino-1-phenylethylamine, 2-dimethylamino-1-phenylethylamine, 2-diethylamino-1-phenylethylamine, 2-di-n-propylamino-1-phenylethylamine, 2-di-i-propylamino-1-phenylethylamine, 2-di-n-butylamino-1-phenylethylamine, 2-di-tert-butylamino-1-phenylethylamine, 2-pyrrolidinyl-1-phenylethylamine, 2-piperidino-1-phenylethylamine, etc, which are optically active.

Further, as the optically active diamine compounds usable in the present invention, examples thereof include optically active diamine compounds described in JP-A 8-225466, JP-A 11-189600, JP-A 2001-58999, JP-A 2002-284790, JP-A 2005-68113, WO 2002/055477, WO 2004/007506, etc.

As preferred examples of the ruthenium complex represented by the general formula (11), the following ones are mentioned. Specifically, the following compounds are mentioned.
(i) W is chlorine atom, bromine atom or iodine atom, Z is a trialkylamine, m = p = s = 1, n = r = 2, q = 0; (ii) W is chlorine atom, bromine atom or iodine atom, Z is pyridyl group or a ring-substituted pyridyl group, m = n = r = s = 1, p = 2, q = 0; (iii) W is an acyloxy group, m = n = r = 1, p = 2, q = s = 0; (iv) W is chlorine atom, bromine atom or iodine atom, Z is dimethylformamide or dimethylacetamide, m = n = r = 1, p = 2, q = 0, and s is an integer of from 0 to 4; (v) W is chlorine atom, bromine atom or iodine atom, U is chlorine atom, bromine atom or iodine atom, Z is a dialkylammonium ion, m = n = p = 2, q = 3, r = s = 1; (vi) W is chlorine atom, bromine atom or iodine atom, U is a neutral ligand of an aromatic compound or an olefin, Z is chlorine atom, bromine atom, iodine atom, I₃, BF₄, ClO₄, OTf, PF₆, SbF₆ or BPh₄, m = n = p = q = r = s = 1; (vii) Z is BF₄, ClO₄, OTf, PF₆, SbF₆ or BPh₄, m = n = r = 1, p = q = 0, s = 2; (viii) W and U may be the same or different, representing hydrogen atom, chlorine atom, bromine atom, iodine atom, a carboxyl group or any other anion group, Z is a diamine compound, m = n = p = q = r = s = 1; (ix) W is hydrogen atom, U is chlorine atom, bromine atom or iodine atom, m = p = q = r = 1, n = 2, s = 0; (x) W is hydrogen atom, Z is BF₄, ClO₄, OTf, PF₆, SbF₆ or BPh₄, m = n = p = r = s = 1, q = 0; (xi) W is chlorine atom, bromine atom or iodine atom, U is a monovalent phosphine ligand, Z is chlorine atom, bromine atom or iodine atom, m = n = p = q = 1, r = z =2; (xii) W and U are the same or different, each representing chlorine atom, bromine atom or iodine atom, m = n = p = q = r = 1, s = 0.

The production method for the ruthenium phosphine complex (11) is not specifically defined. For example, the complex may be produced according to the method to be mentioned below or according to a method similar thereto. In the formulae of the transition metal phosphine complexes to be shown below, cod means 1,5-cyclooctadiene, nbd means norbornadiene, Ph means phenyl group, Ac means acetyl group, acac means acetylacetonate, dmf means dimethylformamide, en means ethylenediamine, DPEN means 1,2-diphenylethylenediamine, DAIPEN means 1,1-di(p-methoxyphenyl)-2-isopropylethylenediamine, MAE means methylaminoethylamine, EAE means ethylaminoethylamine, MAPE means 2-methylamino-1-phenylethylamine, EAPE means 2-ethylamino-1-phenylethylamine, DMAPE means 2-dimethylamino-1-phenylethylamine, DEAPE means 2-diethylamino-1-phenylethylamine, DBAE means di-n-butylaminoethylamine, DBAPE means 2-di-n-butylamino-1-phenylethylamine.

### Ruthenium Complex:

As a method for producing a ruthenium complex, for example, the complex can be produced by heating and refluxing [(1,5-cyclooctadiene)dichlororuthenium] ([Ru(cod)Cl₂]ₙ) and an optically active bidentate phosphine ligand, in an organic solvent in the presence of a trialkylamine, according to the description in literature (J. Chem. Soc., Chem. Commun., p. 922, 1985). In addition, the complex can also be produced by heating and refluxing bis[dichloro(benzene)ruthenium] ([Ru(benzene)Cl₂]₂) and an optically active bidentate phosphine ligand, in an organic solvent in the presence of a dialkylamine, according to the method described in JP-A 11-269185.

Further, the complex can be produced by heating and stirring bis[diiodo(para-cymene)ruthenium] ([Ru(p-cymene)I₂]₂) and an optically active bidentate phosphine ligand in an organic solvent according to the method described in literature (J. Chem. Soc., Chem. Commun., p. 1208, 1989). Further, the complex can be synthetized by reacting Ru₂Cl₄(L)₂NEt₃, which is obtained according to the method in literature (J. Chem. Soc., Chem. Commun., p. 992, 1985) and a diamine compound in an organic solvent, according to the method described in JP-A 11-189600.

As specific examples of the ruthenium complex, for example, the following are mentioned.

Ru(OAc)₂(L), Ru(OCOCF₃)₂(L), Ru₂Cl₄(L)₂NEt₃, [RuCl₂(L)(dmf)ₙ], RuHCl(L), RuHBr(L), RuHI(L), [{RuCl(L)}₂(µ-Cl)₃][Me₂NH₂], [{RuBr(L)}₂(µ-Br)₃][Me₂NH₂], [{RuI(L)}₂(µ-I)₃][Me₂NH₂], [{RuCl(L)}₂(µ-Cl)₃][Me₂NH₂], [{RuBr(L)}₂(µ-Br)₃][Me₂NH₂], [{RuBr(L)}₂(µ-I)₃][Me₂NH₂], [RuCl[PPh₃](L)]₂(µ-Cl)₂, [RuBr[PPh₃](L)]₂(µ-Br)₂, [RuI[PPh₃](L)]₂(µ-I)₂, RuCl₂(L), RuBr₂(L), RuI₂(L), [RuCl₂(L)](dmf)ₙ, RuCl₂(L)(pyridine)₂, RuBr₂(L)(pyridine)₂, RuI₂(L)(pyridine)₂, RuCl₂(L)(2,2'-dipyridine), RuBr₂(L)(2,2'-dipyridine), RuI₂(L)(2,2'-dipyridine), [RuCl(benzene)(L)]Cl, [RuBr(benzene)(L)]Br, [RuI(benzene)(L)]I, [RuCl(p-cymene)(L)]Cl, [RuBr(p-cymene)(L)]Br, [RuI(p-cymene)(L)]I, [RuI(p-cymene)(L)]I₃, [Ru(L)](OTf)₂, [Ru(L)](BF₄)₂, [Ru(L)](ClO₄)₂, [Ru(L)](SbF₆)₂, [Ru(L)](PF₆)₂, [Ru(L)](BPh₄)₂, [RuCl₂(L)](en), [RuBr₂(L)](en), [RuI₂(L)](en), [RuH₂(L)](en), [RuCl₂(L)](DPEN), [RuBr₂(L)](DPEN), [RuI₂(L)](DPEN), [RuH₂(L)](DPEN), [RuCl₂(L)](DAIPEN), [RuBr₂(L)](DAIPEN), [RuI₂(L)](DAIPEN), [RuH₂(L)](DAIPEN), [RuCl₂(L)](MAE), [RuBr₂(L)](MAE), [RuI₂(L)](MAE), [RuH₂(L)](MAE), [RuCl₂(L)](EAE), [RuBr₂(L)](EAE), [RuI₂(L)](EAE), [RuH₂(L)](EAE), [RuCl₂(L)](MAPE), [RuBr₂(L)](MAPE), [RuI₂(L)](MAPE), [RuH₂(L)](MAPE), [RuCl₂(L)](DMAPE), [RuBr₂(L)](DMAPE), [RuI₂(L)](DMAPE), [RuH₂(L)](DMAPE), [RuCl₂(L)](DBAE), [RuBr₂(L)](DBAE), [RuI₂(L)](DBAE), [RuH₂(L)](DBAE), [RuCl₂(L)](DBAPE), [RuBr₂(L)](DBAPE), [RuI₂(L)](DBAPE), [RuH₂(L)](DBAPE), etc.

More preferred catalysts for the asymmetric isomerization in the present invention are complexes containing ruthenium and an optically active bidentate phosphine ligand. Most preferred are the following:
Ru₂Cl₄(L)₂NEt₃, [RuCl₂(L)(dmf)ₙ], [{RuCl(L)}₂(µ-Cl)₃][Me₂NH₂], [{RuBr(L)}₂(µ-Br)₃][Me₂NH₂], [{RuI(L)}₂(µ-I)₃][Me₂NH₂], [{RuCl(L)}₂(µ-Cl)₃][Me₂NH₂], [{RuBr(L)}₂(µ-Br)₃][Me₂NH₂], [{RuBr(L)}₂(µ-I)₃][Me₂NH₂], [RuCl(benzene)(L)]Cl, [RuBr(benzene)(L)]Br, [RuI(benzene)(L)]I, [RuCl(p-cymene)(L)]Cl, [RuBr(p-cymene)(L)]Br, [RuI(p-cymene)(L)]I, [RuI(p-cymene)(L)]I₃, [RuCl₂(L)](MAE), [RuBr₂(L)](MAE), [RuI₂(L)](MAE), [RuH₂(L)](MAE), [RuCl₂(L)](EAE), [RuBr₂(L)](EAE), [RuI₂(L)](EAE), [RuH₂(L)](EAE), [RuCl₂(L)](MAPE), [RuBr₂(L)](MAPE), [RuI₂(L)](MAPE), [RuH₂(L)](MAPE), [RuCl₂(L)](DMAPE), [RuBr₂(L)](DMAPE), [RuI₂(L)](DMAPE), [RuH₂(L)](DMAPE), [RuCl₂(L)](DBAE), [RuBr₂(L)](DBAE), [RuI₂(L)](DBAE), [RuH₂(L)](DBAE), [RuCl₂(L)](DBAPE), [RuBr₂(L)](DBAPE), [RuI₂(L)](DBAPE), [RuH₂(L)](DBAPE), etc.

As the base for use in the present invention, for example, salts represented by the following formula (14) is preferably used.

M'X (14)

In the formula (14), M' represents a metal of Li, Na or K, X represents a halogen atom of Cl, Br or I.

Concretely, for example, preferred here is use of metal salts such as LiCl, LiBr, LiI, NaCl, NaBr, NaI, KCl, KBr or KI. Further, ammonium salts such as (Bn)Et₃NCl, (Bn)Et₃NBr, (Bn)Et₃NI or the like may be selected; and phsohonium salts such as BuPh₃PCl, BuPh₃PBr, BuPh₃PI, (C₆H₁₃)Ph₃PBr, BrPPh₃(CH₂)₄PPh₃Br or the like may be selected to realize high selectivity (Bn is benzyl group, Et is ethyl group, Ph is phenyl group, Bu is butyl group).

As the optically active bidentate phosphine ligand for use in the present invention, there are cases of an (S)-form and an (R)-form, and any of these may be selected in accordance with the absolute configuration of the intended optically active citronellal. Specifically, when geraniol is used as the substance and, for example, when Tol-BINAP is used as the ligand, then an (S)-form of Tol-BINAP may be used for obtaining an (R)-form of citronellal, and an (R)-form of Tol-BINAP may be used for obtaining an (S)-form of citronellal. On the other hand, when nerol is used as the substance, an (S)-form of Tol-BINAP may be used for obtaining an (S)-form of citronellal and an (R)-form of Tol-BINAP may be used for obtaining an (R)-form of citronellal.

Further, in the present invention, an optically active nitrogen compound is used in combination with the optically active bidentate phosphine ligand. As the optically active nitrogen compound, there are cases of an (S)-form and an (R)-form, and any of these may be selected in accordance with the absolute configuration of the intended optically active citronellal.

The amount of the transition metal-optically active phosphine complex to be used is preferably from about 1/100 to 1/50000 mol relative to geraniol (1a) or nerol (1b).

The amount of the base to be added is preferably from 0.5 to 100 equivalents, more preferably from 2 to 40 equivalents relative to the transition metal-optically active phosphine complex.

The reaction solvent may be any one capable of solubilizing the asymmetric isomerization raw material (1) and the catalyst system. For example, usable examples thereof include aromatic hydrocarbon solvents such as toluene and xylene; aliphatic hydrocarbon solvents such as pentane and hexane; halogen-containing hydrocarbon solvents such as methylene chloride; ether solvents such as diethyl ether, diisopropyl ether, diethylene glycol dimethyl ether, tetrahydrofuran and 1,3-dioxolane; alcohol solvents such as methanol, ethanol, 2-propanol, butanol and benzyl alcohol; hetero atom-containing organic solvents such as acetonitrile, DMF and DMSO. Preferred are alcohol solvents. The amount of the solvent may be determined depending on the solubility of the reaction substance and the economic potential thereof. For example, depending on the substance, the reaction may be carried out at a low solvent concentration of 1% or less, or nearly in the absence of a solvent. Preferably, the solvent is used in an amount of from 0.1 to 5.0 times by volume.

The reaction temperature may be from 0 to 150°C but is more preferably within a range of from 100 to 70°C. Regarding the reaction time, the reaction may be finished in a few minutes to 30 hours. After the reaction, the system may be post-processed in an ordinary manner to isolate the intended optically active citronellal.

After the reaction, the intended product may be isolated according to ordinary post- treatment and, if desired, according to a method of distillation, column chromatography, etc. The reaction mode in the present invention may be any of a batch system or a continuous system.

### <Step B>

In the step B in the scheme 1 in the present invention, the optically active citronellal obtained in the step A is cyclized, thereby giving an optically active isopulegol.

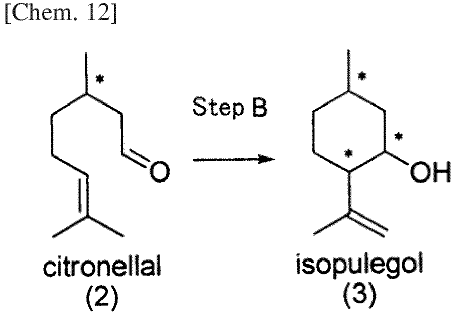

### <Step B: Cyclization catalyst for optically active citronellal>

### (Aluminum Catalyst)

### Step B in Scheme 1:

As the cyclization catalyst for citronellal, an aluminum catalyst is preferably used. The aluminum catalyst may be prepared by reacting an aluminum compound and a phenol ligand.

The aluminum compound to be used for producing the aluminum catalyst is preferably selected from at least one aluminum compound selected from an alkylaluminum compound represented by the general formula (7), a hydride aluminum compound represented by the general formula (8), a linear aluminoxane represented by the general formula (9) and a cyclic aluminoxane represented by the general formula (10).

AlHₖ(Lg)₃₋ₖ (7)

In the formula (7), A1 represents aluminum, Lg represents a branched or linear alkyl group having from 1 to 8 carbon atoms, a cyclic alkyl group having from 5 to 8 carbon atoms, or an aralkyl group having from 7 to 12 carbon atoms, which may have a substituent; and k indicates an integer of from 0 to 3.

MAlH₄ (8)

In the formula (8), A1 represents aluminum, M represents lithium, sodium or potassium.

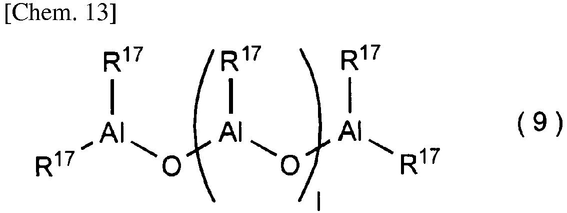

In the formula (9), A1 represents aluminum, R¹⁷ represents a branched or linear alkyl group having from 1 to 8 carbon atoms, a cyclic alkyl group having from 5 to 8 carbon atoms, or an aralkyl group having from 7 to 12 carbon atoms, which may have a substituent, and a plurality of R¹⁷ may be the same or different; and 1 indicates an integer of from 0 to 40.

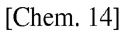

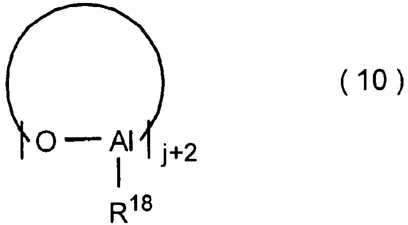

In the formula (10), A1 represents aluminum, R¹⁸ represents a branched or linear alkyl group having from 1 to 8 carbon atoms, a cyclic alkyl group having from 5 to 8 carbon atoms, or an aralkyl group having from 7 to 12 carbon atoms, which may have a substituent; and j indicates an integer of from 0 to 40.

Examples of the substituent in the aluminum compounds represented by the above general formulae (7), (9) and (10) are mentioned below.

Examples of the branched or linear alkyl group having from 1 to 8 carbon atoms include, for example, methyl group, ethyl group, n-propyl group, isopropyl group, n-butyl group, isobutyl group, sec-butyl group, tert-butyl group, pentyl group, hexyl group, heptyl group, octyl group, etc.

Examples of the cyclic alkyl group having from 5 to 8 carbon atoms include, for example, cyclopentyl group, cyclohexyl group, cycloheptyl group, cyclooctyl group, etc.

Examples of the aralkyl group having from 7 to 12 carbon atoms, which may have a substituent, include, for example, benzyl group, 1-phenylethyl group, 2-phenylethyl group, α-naphthylmethyl group, β-naphthylmethyl group, etc.

Examples of the substituent include, for example, an alkyl group having from 1 to 6 carbon atoms such as methyl group, ethyl group, n-propyl group, isopropyl group, n-butyl group, isobutyl group, sec-butyl group, tert-butyl group, pentyl group and a hexyl group; an alicyclic group having from 5 to 8 carbon atoms such as cyclopentyl group, cyclohexyl group and cycloheptyl group; a perfluoroalkyl group having from 1 to 4 carbon atoms such as trifluoromethyl group, pentafluoroethyl group, heptafluoropropyl group and nonafluorobutyl group; an alkoxy group having from 1 to 4 carbon atoms such as methoxy group, ethoxy group, n-propoxyl group, isopropoxy group, n-butoxy group, isobutoxy group, sec-butoxy group and tert-butoxy group; a halogen atom such as fluorine atom, chlorine atom, bromine atom and iodine atom; an aralkyl group having from 7 to 12 carbon atoms such as benzyl group, phenylethyl group and naphthylmethyl group; a tri-C₁₋₆ alkylsilyl group such as trimethylsilyl group, triethylsilyl group, triisopropylsilyl group, dimethylisopropylsilyl group, diethylisopropylsilyl group, dimethyl(2,3-dimethyl-2-butyl)silyl group, tert-butyldimethylsilyl group and dimethylhexylsilyl group; a dialkylamino group having from 2 to 8 carbon atoms such as dimethylamino group, diethylamino group and dibutylamine group, etc.

In addition, 1 and j each indicates an integer of from 0 to 40, preferably an integer of from 2 to 30.

The aluminum compounds represented by the general formulae (9) and (10) are compounds referred to as an aluminoxane. Of the aluminoxanes, preferred are methylaluminoxane, ethylaluminoxane, isobutylaluminoxane and methylisobutylaluminoxane; and more preferred is methylaluminoxane. Different types of above aluminoxane can be used as combined in the groups and between the groups. The above aluminoxane may be prepared under various known conditions.

The phenol ligand to be used for producing the aluminum catalyst is preferably at least one phenol ligand selected from hydroxy compounds (phenol ligands) represented by the general formula (5) and/or hydroxy compounds (bis(diarylphenol) ligands) represented by the general formula (6).

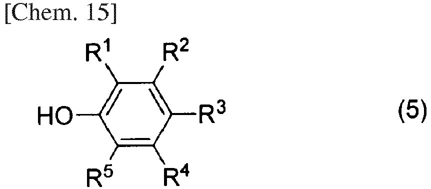

In the formula (5), R¹ and R⁵ each independently represents an aryl group having from 6 to 15 carbon atoms, which may have a substituent, a heteroaryl group having from 4 to 15 carbon atoms, which may have a substituent, or a cyclic alkyl group having from 3 to 15 carbon atoms, which may have a substituent; R², R³ and R⁴ each independently represents a hydrogen atom, an alkyl group having from 1 to 8 carbon atoms, an alkoxy group having from 1 to 8 carbon atoms, an aryl group having from 6 to 15 carbon atoms, which may have a substituent, a perfluoroalkyl group having from 1 to 4 carbon atoms, an aralkyl group having from 7 to 12 carbon atoms, a halogen atom, an organosilyl group, a dialkylamino group having from 1 to 4 carbon atoms, a thioalkoxy group having from 1 to 4 carbon atoms, a nitro group, or a polymer chain.

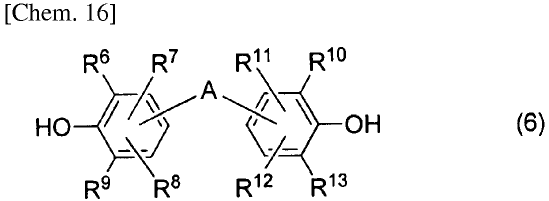

In the formula (6), R⁶, R⁹, R¹⁰ and R¹³ each independently represents an aryl group having from 6 to 15 carbon atoms, which may have a substituent, a heteroaryl group having from 4 to 15 carbon atoms, which may have a substituent, or a cyclic alkyl group having from 3 to 15 carbon atoms, which may have a substituent; R⁷, R⁸, R¹¹ and R¹² each independently represents a hydrogen atom, an alkyl group having from 1 to 8 carbon atoms, an alkoxy group having from 1 to 8 carbon atoms, an aryl group having from 6 to 15 carbon atoms, which may have a substituent, a perfluoroalkyl group having from 1 to 4 carbon atoms, an aralkyl group having from 7 to 12 carbon atoms, a halogen atom, an organosilyl group, a dialkylamino group having from 1 to 4 carbon atoms, a thioalkoxy group having from 1 to 4 carbon atoms, a nitro group, or a polymer chain; A represents (i) a single bond, (ii) a linear, branched or cyclic alkylene group having from 1 to 25 carbon atoms, which may have one or more of a substituent and an unsaturated bond, (iii) an arylene group having from 6 to 15 carbon atoms, which may have a substituent, (iv) a heteroarylene group having from 2 to 15 carbon atoms, which may have a substituent, or (v) a functional group or a hetero element, which is selected from the group consisting of -O-, -S-, -N(R¹⁴)-, -S(O)-, -C(O)-, -S(O)₂-, -P(R¹⁴)-, -(R¹⁴)P(O)- and -Si(R¹⁵R¹⁶)-; R¹⁴ to R¹⁶ each independently represents an alkyl group having from 1 to 6 carbon atoms, a cyclic alkyl group having from 5 to 8 carbon atoms, an aralkyl group having from 7 to 12 carbon atoms, which may have a substituent, or an aryl group having from 6 to 10 carbon atoms, which may have a substituent.

Examples of the specific functional groups in the phenol ligands represented by the above general formula (5) or (6) are mentioned below.

Examples of the aryl group having from 6 to 15 carbon atoms represented by R⁶ to R¹³ include, for example, phenyl group, α-naphthyl group, β-naphthyl group, etc. The aryl group having from 6 to 15 carbon atoms may have a substituent to be mentioned below.

Examples of the heteroaryl group having from 4 to 15 carbon atoms, which is represented by R⁶, R⁹, R¹⁰ and R¹³, include, for example, furyl group, thienyl group, pyronyl group, benzofuryl group, isobenzofuryl group, benzothienyl group, indolyl group, isoindolyl group, carbazolyl group, pyridyl group, quinolyl group, isoquinolyl group, pyrazyl group, ferrocenyl group, etc. The heteroaryl group having rom 4 to 15 carbon atoms may have a substituent to be mentioned below.

Examples of the cyclic alkyl group having from 3 to 15 carbon atoms, which is represented by R⁶, R⁹, R¹⁰ and R¹³ include, for example, cyclopentyl group, cyclohexyl group, cycloheptyl group, cyclooctyl group, cyclodecyl group, cyclododecyl group, norbornyl group, tricyclo[6.2.1.0^{2,7}]-4-undecyl group, etc. The cyclic alkyl group having from 3 to 15 carbon atoms may have a substituent to be mentioned below.

Examples of the alkyl group having from 1 to 8 carbon atoms, which is represented by R⁷, R⁸, R¹¹ and R¹², include, for example, methyl group, ethyl group, n-propyl group, isopropyl group, n-butyl group, isobutyl group, sec-butyl group, tert-butyl group, pentyl group, hexyl group, heptyl group, octyl group, etc.

Examples of the alkoxy group having from 1 to 8 carbon atoms, which is represented by R⁷, R⁸, R¹¹ and R¹², include, for example, methoxy group, ethoxy group, n-propoxyl group, isopropoxy group, n-butoxy group, isobutoxy group, sec-butoxy group, tert-butoxy group, pentoxy group, hexoxy group, heptoxy group, octoxy group, etc.

Examples of the perfluoroalkyl group having from 1 to 4 carbon atoms, which is represented by R⁷, R⁸, R¹¹ and R¹², include, for example, trifluoromethyl group, pentafluoroethyl group, heptafluoropropyl group, nonafluorobutyl group, etc.

Examples of the aralkyl group having from 7 to 12 carbon atoms, which is represented by R⁷, R⁸, R¹¹ and R¹², include, for example, benzyl group, 1-phenylethyl group, 2-phenylethyl group, α-naphthylmethyl group, β-naphthylmethyl group, etc. The aralkyl group having from 7 to 12 carbon atoms may have a substituent to be mentioned below.

Examples of the halogen atom which is represented by R⁷, R⁸, R¹¹ and R¹² include, for example, fluorine atom, chlorine atom, bromine atom, iodine atom, etc.

Examples of the organosilyl group which is represented by R⁷, R⁸, R¹¹ and R¹² include, for example, a tri-substituted silyl group. The substituents for the tri-substituted group are three substituents selected from an alkyl group having from 1 to 6 carbon atoms, an aryl group having from 6 to 18 carbon atoms, and an aralkylsilyl group having from 7 to 19 carbon atoms, and these may be the same or different.

Here, examples of the alkyl group having from 1 to 6 carbon atoms include, for example, methyl group, ethyl group, isopropyl group, 2,3-dimethyl-2-butyl group, hexyl group and tert-butyl group. Examples of the aryl group having from 6 to 18 carbon atoms include, for example, phenyl group and naphthyl group. Examples of the aralkyl group having from 7 to 19 carbon atoms include, for example, benzyl group and p-xylyl group.

Examples of the organosilyl group which is represented by R⁷, R⁸, R¹¹ and R¹² include tri-substituted silyl groups, for example, a tri-C₁₋₆ alkylsilyl group such as trimethylsilyl group, triethylsilyl group, triisopropylsilyl group, dimethylisopropylsilyl group, diethylisopropylsilyl group, dimethyl(2,3-dimethyl-2-butyl)silyl group, tert-butyldimethylsilyl group and dimethylhexylsilyl group; a di-C₁₋₆ alkyl-C₆₋₁₈ arylsilyl group such as dimethylcumylsilyl group, a di-C₆₋₁₈ aryl-C₁₋₆ alkylsilyl group such as tert-butyldiphenylsilyl group and diphenylmethylsilyl group; a tri-C₆₋₁₈ arylsilyl group such as triphenylsilyl group; a tri-C₇₋₁₉ aralkylsilyl group such as tribenzylsilyl group and tri-p-xylylsilyl group, etc.

Examples of the dialkylamino group having from 1 to 4 carbon atoms, which is represented by R⁷, R⁸, R¹¹ and R¹², include, for example, dimethylamino group, diethylamino group, dipropylamino group, diisopropylamino group, dibutylamino group, etc.

Examples of the thioalkoxy group having from 1 to 4 carbon atoms, which is represented by R⁷, R⁸, R¹¹ and R¹², include, for example, methylthio group, ethylthio group, n-propylthio group, isopropylthio group, n-butylthio group, isobutylthio group, sec-butylthio group, tert-butylthio group, etc.

Examples of the polymer chain which is represented by R⁷, R⁸, R¹¹ and R¹² include, for example, a 6,6-nylon chain, a vinyl polymer chain, a styrene polymer chain, etc.

Here, examples of the substituent in R⁶ to R¹³ include an alkyl group having from 1 to 6 carbon atoms such as methyl group, ethyl group, n-propyl group, isopropyl group, n-butyl group, isobutyl group, sec-butyl group, tert-butyl group, pentyl group and hexyl group; a cyclic alkyl group having from 5 to 12 carbon atoms such as cyclopentyl group, cyclohexyl group, cycloheptyl group and cyclooctyl group; a perfluoroalkyl group having from 1 to 4 carbon atoms such as trifluoromethyl group, pentafluoroethyl group, heptafluoropropyl group and nonafluorobutyl group; an alkoxy group having from 1 to 4 carbon atoms such as methoxy group, ethoxy group, n-propoxyl group, isopropoxy group, n-butoxy group, isobutoxy group, sec-butoxy group and tert-butoxy group; a halogen atom such as fluorine atom, chlorine atom, bromine atom and iodine atom; an aralkyl group having from 7 to 12 carbon atoms such as benzyl group, phenylethyl group and naphthylmethyl group; a tri-(C₁₋₆)alkylsilyl group such as trimethylsilyl group, triethylsilyl group, triisopropylsilyl group, dimethylisopropylsilyl group, diethylisopropylsilyl group, dimethyl(2,3-dimethyl-2-butyl)silyl group, tert-butyldimethylsilyl group and dimethylhexylsilyl group; a dialkylamino group having from 2 to 8 carbon atoms such as dimethylamino group, diethylamino group, dibutylamino group and the like. Further examples thereof include polymer chains such as 6,6-nylon chain, vinyl polymer chain and styrene polymer chain.

In the general formula (6), R⁷ or R⁸ and/or R¹¹ or R¹² may form, together with the structural element A, a cyclic aromatic ring or a non-aromatic ring. In this case, the bis(diarylphenol) ligand represented by the general formula (6) for use in the present invention has a tricyclic basic structure, for example, an anthracene basic structure having a formula (X) or a basic structure of the type (Y).

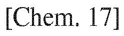

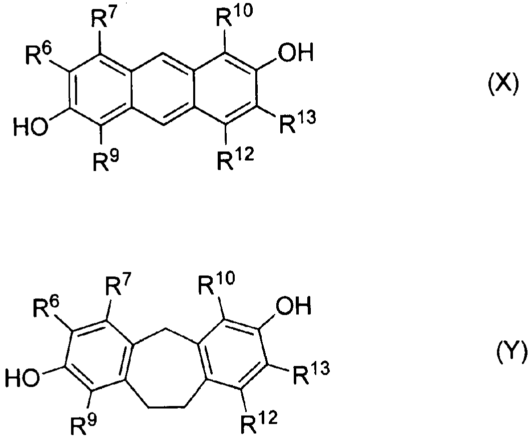

In the formulae (X) and (Y), R⁶, R⁷, R⁹, R¹⁰, R¹² and R¹³ are the same as above.

If appropriate, any further structural modification of these tricyclic basic structures including those having a hetero atom in the basic structure are known to those skilled in the art, and belongs to the group of bis(diarylphenol)ligand capable of being used according to the present invention.

A in the general formula (6) represents (i) a single bond, (ii) a linear, branched or cyclic alkylene group having from 1 to 25 carbon atoms, which may have one or more of a substituent and an unsaturated bond, (iii) an arylene group having from 6 to 15 carbon atoms, which may have a substituent, (iv) a heteroarylene group having from 2 to 15 carbon atoms, which may have a substituent, or (v) a functional group or a hetero element, which is selected from the group consisting of -O-, -S-, -N(R¹⁴)-, -S(O)-, -C(O)-, -S(O)₂-, -P(R¹⁴)-, -(R¹⁴)P(O)- and -Si(R¹⁵R¹⁶)-. Here, R¹⁴ to R¹⁶ each independently represents an alkyl group having from 1 to 6 carbon atoms, a cyclic alkyl group having from 5 to 8 carbon atoms, an aralkyl group having from 7 to 12 carbon atoms, which may have a substituent, or an aryl group having from 6 to 10 carbon atoms, which may have a substituent.

Examples of the linear, branched or cyclic alkylene group having from 1 to 25 carbon atoms (ii), which is represented by A, include, for example, methylene group, ethylene group, isopropyl group, n-butylene group, isobutylene group, sec-butylene group, tert-butylene group, dodecylene group, undecylene group, cyclopentylene group, cyclohexylene group, cycloheptylene group, cyclooctylene group, cyclodecylene group, cyclododecylene group, norbornylene group, tricyclo[6.2.1.0^{2.7}]-4-undecylene group.

The linear, branched or cyclic alkylene group having from 1 to 25 carbon atoms may have one or more of a substituent and an unsaturated bond. As the substituent, examples thereof include the same substituents as those exemplified as the substituents for the above-mentioned R⁶ to R¹³.

Examples of (iii) the arylene group having from 6 to 15 carbon atoms, which is represented by A, include, for example, phenylene group, naphthylene group, anthracenylene group, etc.

The arylene group having from 6 to 15 carbon atoms may have one or more of a substituent and an unsaturated bond. As the substituent, examples thereof include the same substituents as those exemplified as the substituents for the above-mentioned R⁶ to R¹³.

Examples of (iv) the heteroarylene group having from 2 to 15 carbon atoms, which is represented by A, include, for example, furylene group, thienylene group, pyrrolylene group, benzofurylene group, isobenzofurylene group, benzothienylene group, indolylene group, isoindolylene group, carbazoylene group, pyridylene group, quinolylene group, isoquinolylene group, pyrazylene group, ferrocenylene group, etc.

The heteroarylene group having from 2 to 15 carbon atoms may have one or more of a substituent and an unsaturated bond. As the substituent, examples thereof include the same substituents as those exemplified as the substituents for the above-mentioned R⁶ to R¹³.

A may also be (v) a functional group or a hetero element selected from the group consisting of -O-, -S-, -N(R¹⁴)-, -S(O)-, -C(O)-, -S(O)₂-, -P(R¹⁴)-, -(R¹⁴)P(O)-and -Si(R¹⁵R¹⁶)-. Here, R¹⁴ to R¹⁶ each independently represents at least one group of an alkyl group having from 1 to 6 carbon atoms, a cyclic alkyl group having from 5 to 8 carbon atoms, an aralkyl group having from 7 to 12 carbon atoms, which may have a substituent, or an aryl group having from 6 to 10 carbon atoms, which may have a substituent. As the substituent, examples thereof include the same substituents as those exemplified as the substituents for the above-mentioned R⁶ to R¹³. Here, A is preferably -O-, -S-, -S(O)-, -S(O)₂- or -Si(R¹⁵R¹⁶)-.

As the alkyl group having from 1 to 6 carbon atoms, the cyclic alkyl group having from 5 to 8 carbon atoms, the aralkyl group having from 7 to 12 carbon atoms, which may have a substituent, and the aryl group having from 6 to 10 carbon atoms, which may have a substituent, for R¹⁴ to R¹⁶, examples thereof include the same groups as those exemplified for those for the phenol ligands represented by the above-mentioned general formulae (5) and (6).

As specific examples of A, for example, the following structures are mentioned. The wavy line indicates the bonding site at which the structure bonds to the remaining ligand structure as in the range disclosed in this description.

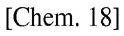

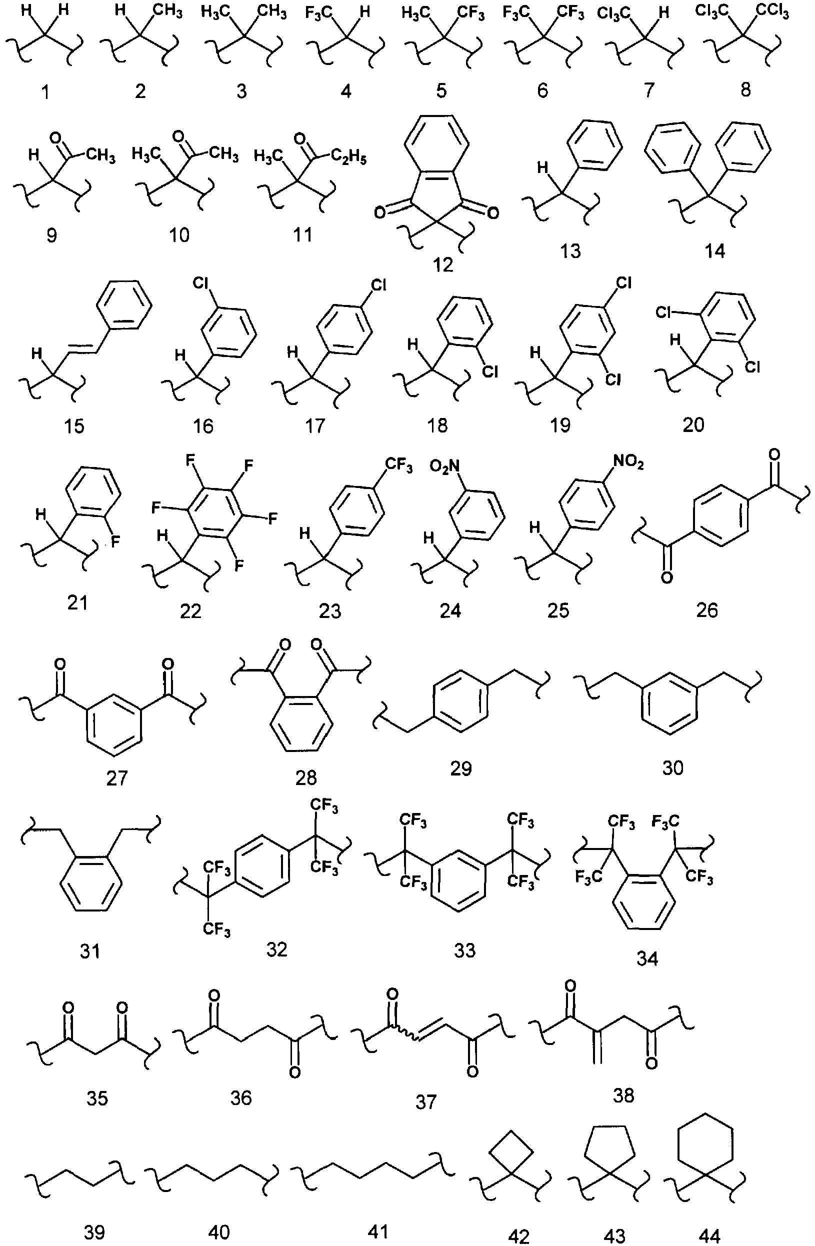

The above-mentioned structures 1 to 44 may have a substituent. Here, examples of the substituent include the same substituents as those exemplified for the aryl group having from 6 to 15 carbon atoms in the phenol ligands represented by the above-mentioned general formula (5) and (6).

The phenol ligands of the general formula (5) are, for example, described in JP-A 2002-212121 (Patent Document 8) [which is incorporated herein by reference].

The bis(diarylphenol) of the general formula (6) are, for example, described in JP-T 2008-538101 (Patent Document 9) [which is incorporated herein by reference].

Preferred phenol ligand in the present invention is at least one phenol ligand selected from phenol ligands represented by the following general formula (5-a) and bis(diarylphenol) ligands represented by the following general formula (6-a).

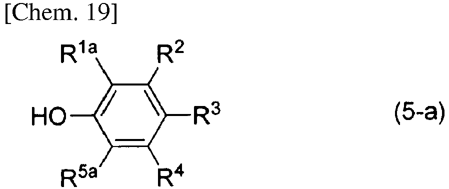

In the formula (5-a), R^{1a} represents an aryl group having from 6 to 15 carbon atoms, which may have a substituent, or a heteroaryl group having from 4 to 15 carbon atoms, which may have a substituent; R^{5a} represents a cyclic alkyl group having from 5 to 15 carbon atoms, which may have a substituent; R², R³ and R⁴ each independently represents a hydrogen atom, an alkyl group having from 1 to 8 carbon atoms, an alkoxy group having from 1 to 8 carbon atoms, an aryl group having from 6 to 15 carbon atoms, which may have a substituent, a perfluoroalkyl group having from 1 to 4 carbon atoms, an aralkyl group having from 7 to 12 carbon atoms, a halogen atom, an organosilyl group, a dialkylamino group having from 1 to 4 carbon atoms, a thioalkoxy group having from 1 to 4 carbon atoms, a nitro group, or a polymer chain.

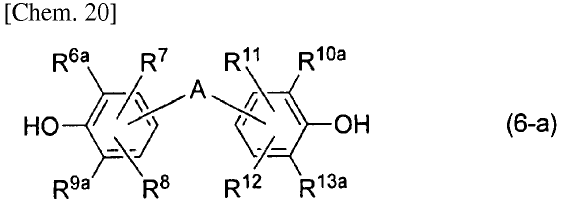

In the formula (6-a), R^{6a} and R^{10a} each independently represents an aryl group having from 6 to 15 carbon atoms, which may have a substituent, or a heteroaryl group having from 4 to 15 carbon atoms, which may have a substituent; R^{9a} and R^{13a} each represents a cyclic alkyl group having from 5 to 15 carbon atoms, which may have a substituent; R⁷, R⁸, R¹¹ and R¹² each represents a hydrogen atom, an alkyl group having from 1 to 8 carbon atoms, an alkoxy group having from 1 to 8 carbon atoms, an aryl group having from 6 to 15 carbon atoms, which may have a substituent, a perfluoroalkyl group having from 1 to 4 carbon atoms, an aralkyl group having from 7 to 12 carbon atoms, a halogen atom, an organosilyl group, a dialkylamino group having from 1 to 4 carbon atoms, a thioalkoxy group having from 1 to 4 carbon atoms, a nitro group, or a polymer chain, A represents (i) a single bond, (ii) a linear, branched or cyclic alkylene group having from 1 to 25 carbon atoms, which may have one or more of a substituent and an unsaturated bond, (iii) an arylene group having from 6 to 15 carbon atoms, which may have a substituent, (iv) a heteroarylene group having from 2 to 15 carbon atoms, which may have a substituent, or (v) a functional group or a hetero element, which is selected from the group consisting of -O-, -S-, -N(R¹⁴)-, -S(O)-, -C(O)-, -S(O)₂-, -P(R¹⁴)-, -(R¹⁴)P(O)- and -Si(R¹⁵R¹⁶)-; R¹⁴ to R¹⁶ each independently represents an alkyl group having from 1 to 6 carbon atoms, a cyclic alkyl group having from 5 to 8 carbon atoms, an aralkyl group having from 7 to 12 carbon atoms, which may have a substituent, or an aryl group having from 6 to 10 carbon atoms, which may have a substituent.

In the present invention, preferred examples of the phenol ligands represented by the general formula (5) include, for example, 2,6-diphenylphenol, 2,6-di(4-chlorophenyl)phenol, 2,6-di(3,4-difluorophenyl)phenol, 2,6-di(3,4,5-trifluorophenyl)phenol, 2,6-diphenyl-4-methylphenol, 2,6-diphenyl-3,5-dimethylphenol, 2,6-di(2-methylphenyl)-3,5-dimethylphenol, 2,6-di(2-isopropylphenyl)-3,5-dimethylphenol, 2,6-di(α-naphthyl)-3,5-dimethylphenol, 3-phenyl-1,1'-binaphthyl-2-ol, 3-(4-fluorophenyl)-1,1'-binaphthyl-2-ol, 1,3-diphenyl-2-naphthol, 3,3',5,5,'-tetraphenylbiphenyl-4,4'-diol, a 2-cycloalkyl-6-arylphenol (general formula (5-a)), etc.

In the present invention, preferred examples of the phenol ligands represented by the general formula (5-a) (a 2-cycloalkyl-6-arylphenol) include, for example, the following structures.

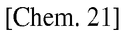

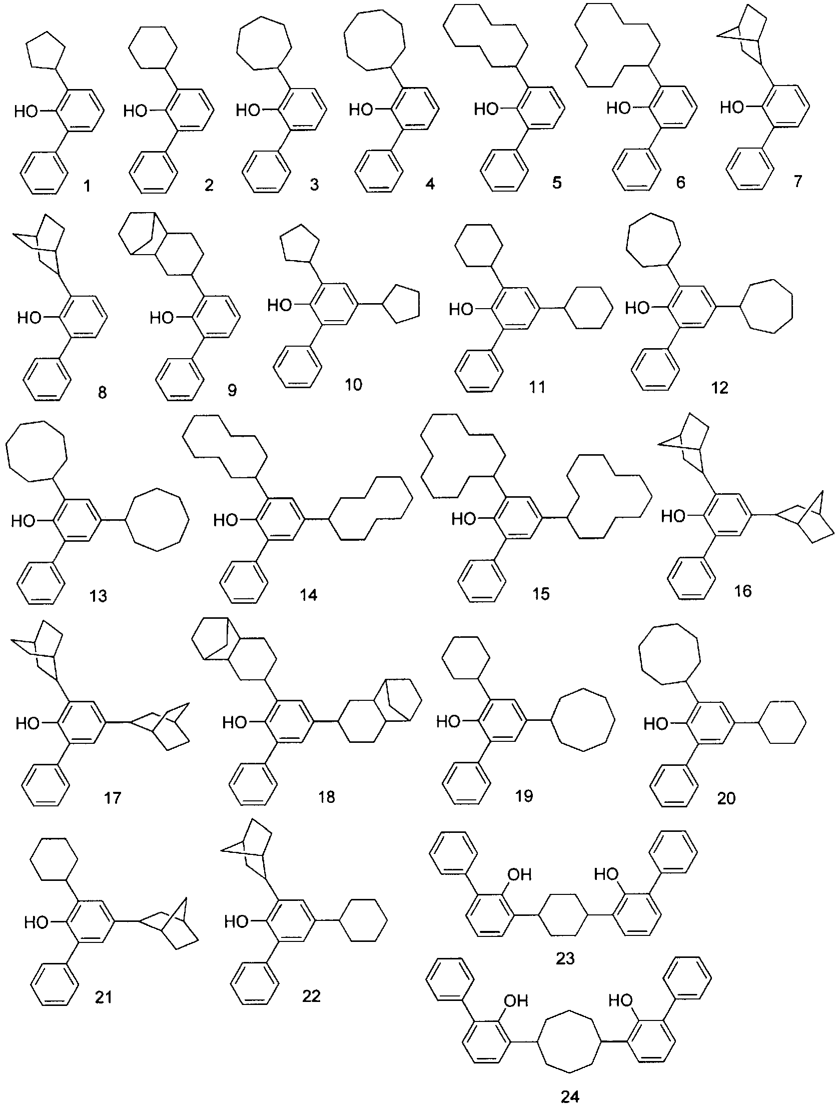

In the present invention, preferred examples of the bis(diarylphenol) ligands represented by the general formula (6) include, for example, the following structures and the bis(2-cycloalkyl-6-arylphenol (general formula (6-a)), etc. (In the formulae, A has the same meaning as above.)

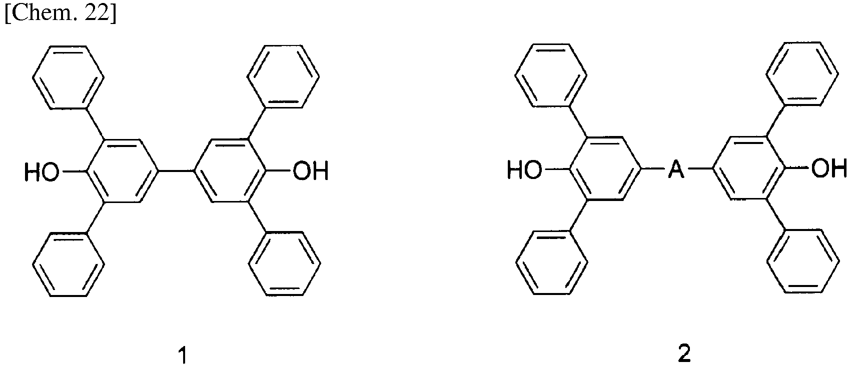

In the present invention, preferred examples of the bis(diarylphenol) ligands represented by the formula (6-a) include, for example, the following structures. (In the formulae, A has the same meaning as above.)

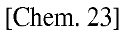

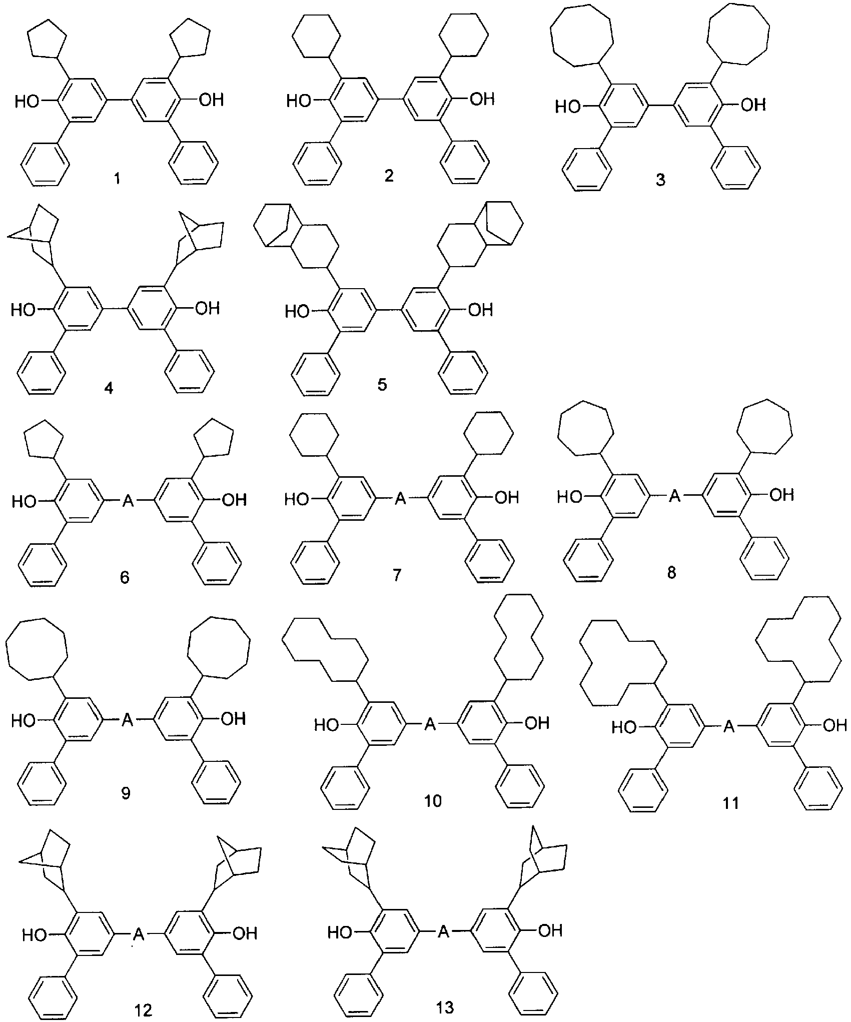

Each of the phenol ligands represented by the above general formulae (5), (5-a), (6) and (6-a) may be synthesized according to conventional synthesis methods, or are generally available compounds.
2-Cyclohexyl-6-phenylphenol that is one ligand of the aluminum catalyst in the present invention is a precursor of 2,6-diphenylphenol which has been employed in the art, and can be produced easily and inexpensively in the presence of an acidic catalyst (JP-A 2009-269868).

The aluminum catalyst in the present invention can be obtained by reacting at least one selected from aluminum compounds represented by the above-mentioned general formulae (7), (8), (9) and (10) and at least one selected from phenol ligands represented by the above-mentioned general formulae (5), (5-a), (6) and (6-a).

In that case, it is desirable that the phenol ligand is reacted with the aluminum compound in a ratio of preferably from 1.0 to 5 equivalents, more preferably from 1.4 to 3.5 equivalents relative to the aluminum compound (as a molar ratio of aluminum atom/compound).

The reaction can be carried out in an inert gas atmosphere or in the presence of an inert solvent.

As the inert gas, for example, preferred is use of nitrogen, argon, other rare gases, etc.

As the inert solvent, for example, examples thereof include aliphatic hydrocarbons (hexane, heptane, octane, etc.), alicyclic hydrocarbons (cyclohexane, methylcyclohexane, etc.), aromatic hydrocarbons (benzene, toluene, xylene, etc.), ethers (diethyl ether, diisopropyl ether, dimethoxyethane, methyl tert-butyl ether, tetrahydrofuran, dioxane, dioxolane, etc.), halogenated hydrocarbons (dichloromethane, dichloroethane, chlorobenzene, etc.), etc. Of those, preferred are organic solvents such as toluene and heptane. Preferably, these solvents are previously-dried ones or anhydrides.

The amount (L) of the solvent to be used is preferably within a range of from 1 to 10000 times by volume [L/kg] relative to the phenol ligand (kg), more preferably from 20 to 400 times by volume [L/kg]. Preferably, the degree of polymerisation of the aluminoxanes represented by the general formulae (9) and (10) is 2 or more.

The reaction temperature is preferably within a range of from about -60 to 100°C, more preferably from about -30 to 50°C, even more preferably from about -10 to 30°C. While keeping at the temperature, the reaction is carried out preferably for about 0.25 to 30 hours, more preferably for about 0.5 to 10 hours to smoothly produce the aluminum catalyst.

The aluminum catalyst in the present invention exhibits excellent catalytic effects in intramolecular reaction, especially in intramolecular cyclization reaction.

The aluminum catalyst in the present invention can be used as a catalyst for cyclization reaction of an optically active citronellal to synthesize an optically active isopulegol.

In the present invention, an optically active citronellal is cyclized in the presence of the above-mentioned catalyst, thereby giving an optically active isopulegol.

The starting compound, i.e. optically active citronellal, for use herein is one produced according to the step A.

The amount of the aluminum catalyst to be used in the cyclization reaction of the optically active citronellal in the step B is preferably within a range of from about 0.05 to 10 mol% relative to citronellal, more preferably within a range of from about 0.1 to 3 mol%.

The aluminum catalyst to be used in the cyclization reaction of the optically active citronellal in the present invention can realize the same result according to any method of a method a) of mixing at least one selected from the aluminum compounds represented by the general formulae (7), (8), (9) and (10) and at least one selected from the phenol ligands represented by the general formulae (5), (5-a), (6) and (6-a) to previously prepare the aluminum catalyst in the reaction system, followed by adding the optically active citronellal to the system, or a method b) of mixing at least one selected from the aluminum compounds and at least one selected from the phenol ligands to previously prepare the aluminum catalyst, and then adding the aluminum catalyst and the optically active citronellal separately to the reactor for cyclization reaction during the course of the reaction.

The temperature of the cyclization reaction of the optically active citronellal is preferably within a range of from about -60 to 60°C, more preferably within a range of from about -30 to 40°C, even more preferably from about -20 to 20°C. While keeping at the temperature, the reaction is carried out preferably for about 0.25 to 30 hours, more preferably for about 0.5 to 20 hours to smoothly produce the optically active isopulegol represented by the formula (3).

Preferably, the cyclization reaction of the optically active citronellal in the present invention is carried out in the absence of a solvent or in the presence of an inert solvent and in an inert gas atmosphere of nitrogen gas or argon gas, and such a condition is preferred for smooth progress of the cyclization reaction.

The solvent to be used may be any solvent not markedly interfering with the reaction and is not specifically defined. Examples thereof include aliphatic hydrocarbons (hexane, heptane, octane, etc.), alicyclic hydrocarbons (cyclohexane, methylcyclohexane, etc.), aromatic hydrocarbons (benzene, toluene, xylene, etc.), ethers (diethyl ether, diisopropyl ether, dimethoxyethane, methyl tert-butyl ether, tetrahydrofuran, dioxane, dioxolane, etc.), halogenated hydrocarbons (dichloromethane, dichloroethane, chlorobenzene), etc. Of those, preferred are organic solvents such as toluene and heptane. Preferably, these solvents are previously-dried ones or anhydrides.

The amount of the solvent to be used is preferably from about 0 to 20 times by volume relative to the optically active citronella, more preferably from 0.5 to 7 times by volume.

During the cyclization reaction, an additive may be added. Specific examples of the additive include, for example, mineral acids (hydrochloric acid, sulfuric acid, etc.), organic acids and their ester compounds (formic acid, acetic acid, pyruvic acid, propionic acid, citronellic acid, geranic acid, neric acid, etc., or their alkyl/aryl esters), aldehydes other than citronellal (chloral, acetaldehyde, p-bromobenzaldehyde, ethyl glyoxylate, etc.), organic acid anhydrides (acetic anhydride, propionic anhydride, decanoic anhydride, maleic anhydride, citronellic anhydride, succinic anhydride, pivalic anhydride, etc.), ketones (perfluoroacetone, 1,1,1-trifluoroacetone, etc.), acid halides (acetic acid chloride, propionic acid chloride, decanoic acid chloride, etc.), vinyl ethers (methyl vinyl ether, ethyl vinyl ether, etc.), epoxy compounds (α-pinene oxide, isobutylene oxide, isopulegol oxide, etc.).

The additive, i.e. acids or their ester compounds, aldehydes other than citronellal, organic acid anhydrides, ketones, acid halides, vinyl ethers and epoxy compounds may be added to the catalyst layer or the citronellal layer in any stage after preparation of the aluminum catalyst to conduct the cyclization reaction of citronellal.

The amount of the acids or their ester compounds, aldehydes, organic acid anhydrides, ketones, acid halides, vinyl ethers and epoxy compounds to be added is preferably from 10 to 100 (% by weight) relative to the aluminum of the cyclization catalyst (weight), more preferably from 20 to 60 (% by weight).

After the reaction, ordinary post-treatment can be carried out. The optically active isopulegol obtained in the step B in the scheme 1 may be purified by mere distillation, or cryogenic crystallization in the step D in the scheme 1, and then, a high-purity optically active isopulegol may be obtained.

The ligand in all the organic aluminum compounds may be recovered after the catalyst deactivation and may be reused in the catalyst. In other words, the residue after the distillation treatment may be processed with an acid or an alkali in an ordinary manner to remove impurities including aluminium and the like, and thereafter, the residue may be processed for crystallization, distillation or the like, and the resultant hydroxy compound may be reused as the phenol ligand.

On the other hand, regarding the aluminum catalyst in the present invention that is hardly soluble in a solvent, the solution after the reaction may be filtered or processed for decantation to remove the formed isopulegol, and then, citronellal may be put thereinto to carry out continuous cyclization reaction. Also, the catalyst may be taken out through filtration after the cyclization reaction, and may be used in the next cyclization reaction directly.

In a case where the aluminum catalyst is partially deactivated, a new catalyst capable of replenishing for the part of the deactivated catalyst may be added to the reaction solution and may be used in the next cyclization reaction.

### <Step D>

The step D in the scheme 1 in the present invention includes crystallizing the optically active isopulegol obtained in the step B, at a low temperature (in a mode of cryogenic crystallization). Accordingly, it is possible to produce an optically active isopulegol having higher chemical purity and higher optical purity.

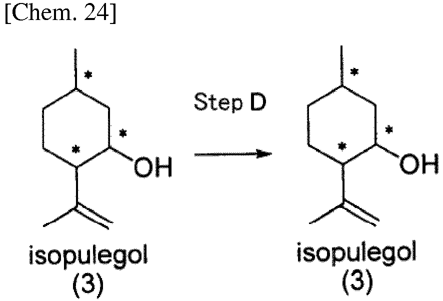

### <Step D: Cryogenic crystallization of optically active isopulegol>

Cryogenic crystallization of the optically active isopulegol is described, for example, in Japanese Patent 3241542 [which is incorporated herein by reference].

A solution prepared by dissolving the optically active isopulegol obtained in the step B in an organic solvent is crystallized at a low temperature (cryogenic crystallization), thereby giving an optically active isopulegol of which both of the chemical purity and the optical purity are 99.7% or more.

The temperature for the cryogenic crystallization of the optically active isopulegol is preferably within a range of from about -60 to -20°C, more preferably within a range of from about -50 to -25°C. Gradually lowering the above-mentioned temperature, an optically active isopulegol of which both of the chemical purity and the optical purity are 99.7% or more is crystallized and ripened with stirring. For promoting the crystallization, a small amount of an optically active isopulegol crystal of which both of the chemical purity and the optical purity are 99.7% or more may be added.

The crystallization time is preferably from about 1 to 30 hours, more preferably from about 10 to 20 hours. Subsequently, the high-purity isopulegol crystallized is filtered with a centrifuge, thereby producing the high-purity optically active isopulegol in the step D in the scheme 1.

The solvent to be used is not specifically defined. Examples thereof include aliphatic hydrocarbons (hexane, heptane, octane, petroleum ether, etc.), alicyclic hydrocarbons (cyclohexane, methylcyclohexane, etc.), aromatic hydrocarbons (benzene, toluene, xylene, etc.), ethers (diethyl ether, diisopropyl ether, dimethoxyethane, methyl tert-butyl ether, tetrahydrofuran, dioxane, dioxolane, etc.), alcohols (methanol, ethanol, isopropanol, etc.), ketones (acetone, methyl ethyl ketone, etc.), or their mixed solvents. Of those, the organic solvents such as heptane, petroleum ether and acetone are preferred. Preferably, these solvents are previously-dried ones or anhydrides.

The amount (L) of the solvent to be used is preferably from 0.5 to 5 times by volume [L/kg] relative to isopulegol (kg), more preferably from 1 to 3 times by volume [L/kg].

The high-purity optically active isopulegol that is odorless and has only refresh feeling can be processed into commercial products through precision distillation with from 5 to 50 theoretical stages, etc. In a case where the optically active isopulegol is processed for precision distillation prior to cryogenic crystallization, it is possible to produce the high-purity optically active isopulegol that is odorless and has only refresh feeling only through simple distillation after the cryogenic treatment.

### <Step C and Step E>

The step C and the step E in the scheme 1 of the present invention includes hydrogenating the optically active isopulegol obtained in the step B or the step D, using a catalyst, thereby giving an optically active menthol.

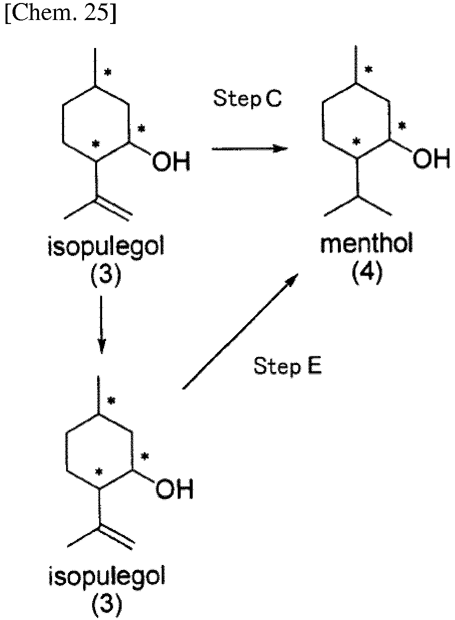

### <Step C and Step E: Hydrogenation of optically active isopulegol>

Hydrogenation of the carbon-carbon double bond part of the optically active isopulegol may be carried out in an ordinary method. Specifically, a catalyst having a hydrogenation capability such as Raney nickel or Pd/C is put into an autoclave, and the optically active isopulegol can be hydrogenated therein under hydrogen pressure in the absence or presence of a solvent, thereby giving an optically active menthol.

The temperature in hydrogenation of the optically active isopulegol is preferably within a range of from about 0 to 80°C, more preferably within a range of from about 20 to 60°C. The reaction time is preferably from about 1 to 30 hours, more preferably from about 3 to 15 hours. Subsequently, the optically active menthol is filtered, and distilled, thereby producing the optically active menthol.

The solvent to be used is not specifically defined. Examples thereof include aliphatic hydrocarbons (hexane, heptane, octane, petroleum ether, etc.), alicyclic hydrocarbons (cyclohexane, methylcyclohexane, etc.), aromatic hydrocarbons (benzene, toluene, xylene, etc.), ethers (diethyl ether, diisopropyl ether, dimethoxyethane, methyl tert-butyl ether, tetrahydrofuran, dioxane, dioxolane, etc.), alcohols (methanol, ethanol, isopropanol, etc.), ketones (acetone, methyl ethyl ketone, etc.), or their mixed solvents. Of those, organic solvents such as heptane, petroleum ether and acetone are preferred. Preferably, these solvents are previously-dried ones or anhydrides.

The amount (L) of the solvent to be used is preferably from 0 to 5 times by volume [L/kg] relative to the optically active menthol (kg), more preferably from 0 to 3 times by volume [L/kg].

### Examples

The present invention is described in detail with reference to the following Comparative Examples and Examples, but the present invention is not whatsoever limited. Within a range not overstepping the scope thereof, the present invention may be changed and modified.

The products in Synthesis Examples and Examples were identified using the following instruments.
Nuclear Magnetic Resonance Spectrum (¹H-NMR): Oxford 300 MHz FT-NMR (300 MHz, solvent CDCl₃) (by Barian, Inc.)
Gas Chromatography: GC-2010 Gas Chromatograph, manufactured by Shimadzu Corporation
Measurement of Additive Rate: DB-WAX (0.25 mm x 30 m), manufactured by Agilent Technologies
Measurement of Optical Purity: beta-DEX-225 (0.25 mm x 30 m), manufactured by Supelco
Detector: FID

### (Example 1) Asymmetric isomerization of geraniol

In an argon stream, a catalyst [A] represented by the general formula (14): RuCl₂[(R)-DBAPE][(S)-TolBINAP]; Ar = 4-CH₃C₆H₄, R = n-C₄H₉, R' = C₆H₅ (3.2 mg, 0.0029 mmol), geraniol (89.3 mg, 0.579 mmol), ethanol (2.6 mL) and 46.7 mM-KOH (0.31 mL, 0.015 mmol) were added and stirred at 25°C for 1 hour.

After the reaction, the solution was concentrated under reduced pressure, and the residue was purified through silica gel short-pass column, thereby giving (R)-citronellal (52.9 mg, 58%). As a result of analysis through gas chromatography (column: BetaDEX225 (0.25 mm x 30 m, DF = 0.25)), the optical purity of the product was 99% e.e. or more.

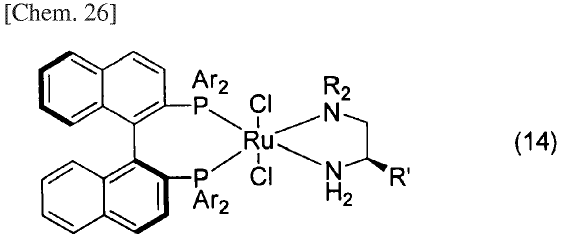

### (Example 2) Asymmetric isomerization of nerol

Nerol, as a starting material, was reacted in the same manner as in Example 1 except that the catalyst used in Example 1, the molar ratio of the substance to the catalyst, the reaction temperature, and the reaction time were changed as in Table 1. The yield of (S)-citronellal was 83% as GC yield. In the same manner as in Example 1, after the reaction, the solvent was concentrated under reduced pressure, and the residue was analyzed through gas chromatography. The optical purity was 99% e.e.

### (Example 3) Asymmetric isomerization of geraniol

In a 3-L reaction flask, the catalyst [A] used in Example 1 (3.2 g, 2.9 mmol), geraniol (89.3 g, 0.579 mol), ethanol (2.6 L), and 46.7 mM-KOH (0.31 L, 0.015 mol) were added, and stirred at 25°C for 1 hour.

After the reaction, the solution was distilled under reduced pressure (69 to 71°C, 0.66 kPa), thereby giving (R)-citronellal (52.9 g, 58%). As a result of analysis through gas chromatography (column; BetaDEX225 (0.25 mm x 30 m, DF = 0.25)), the optical purity of the product was 99% e.e. or more. The reaction was repeated four times.

### (Example 4) Preparation of aluminum catalyst and synthesis of 1-isopulegol

2,6-Diphenylphenol (3.621 g, 14. 7 mmol) was put into a 1-L reaction flask, followed by purging with nitrogen, and toluene (96 mL) and then triethylaluminum toluene solution (1.0 mol/L) (4.54 mL, 4.54 mmol) were added thereto with stirring in the nitrogen atmosphere. After stirred at room temperature for 1 hour, the internal temperature was cooled down to 0 to 10°C, and d-citronellal (100.0 g, 648.3 mmol, optical purity 97.8% e.e.) was dropwise added thereto, followed by stirring overnight at 0 to 10°C. After the reaction, water was added to the solution sample, and the organic layer was analyzed through gas chromatography. As a result, the substance conversion was 99.5%, the isopulegol selectivity was 98.2%, and the ratio of 1-n-isopulegol to the other isomer was 99.5/0.5.

### (Example 5) Preparation of aluminum catalyst and synthesis of 1-isopulegol

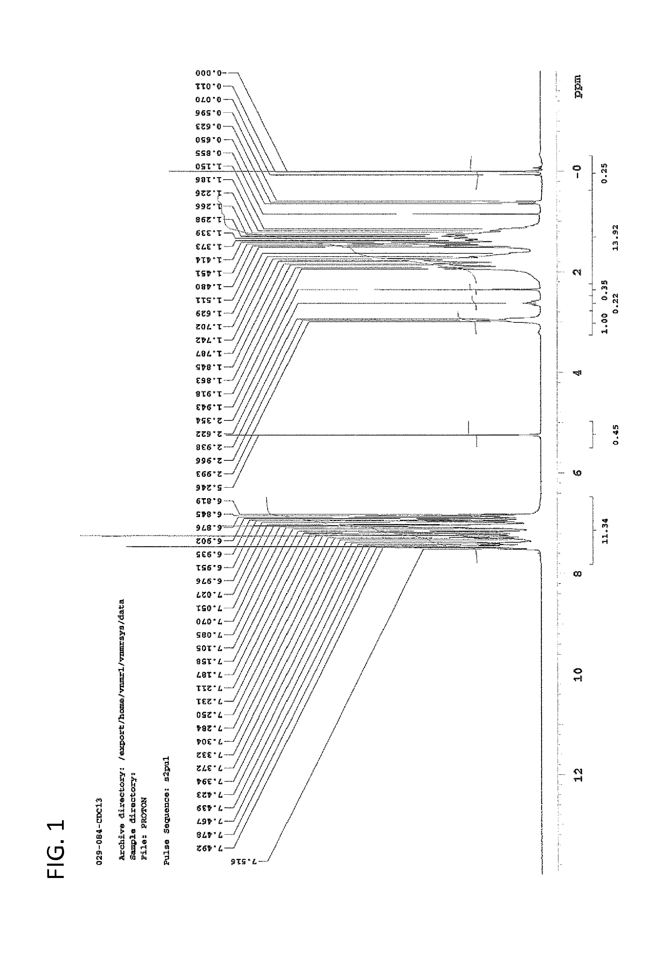

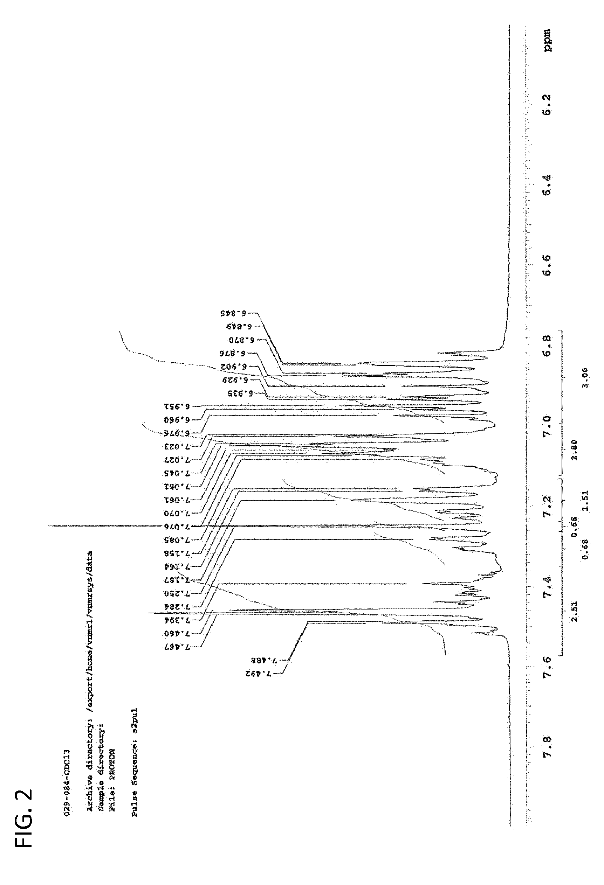

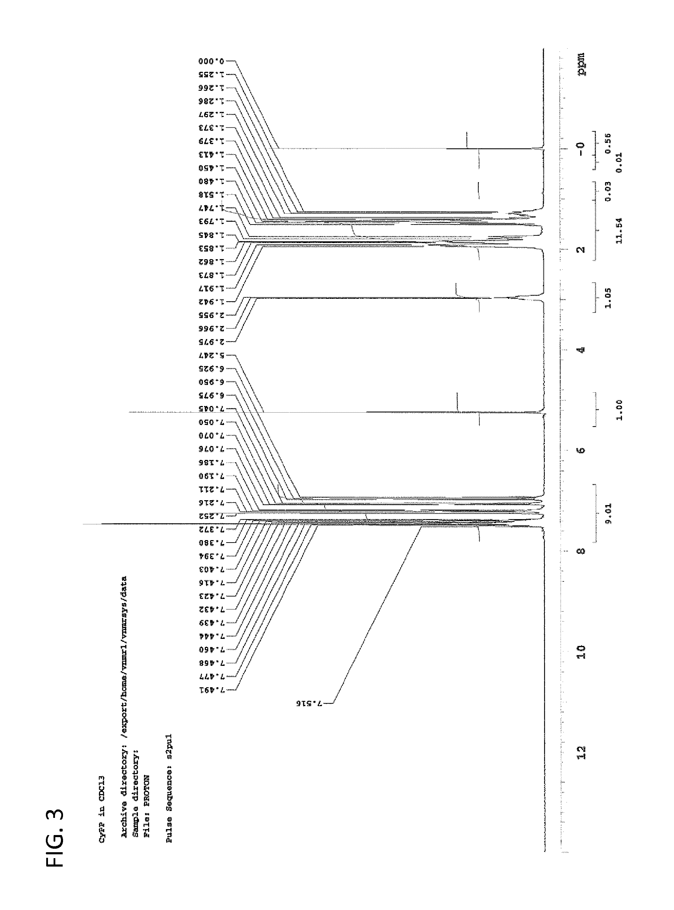

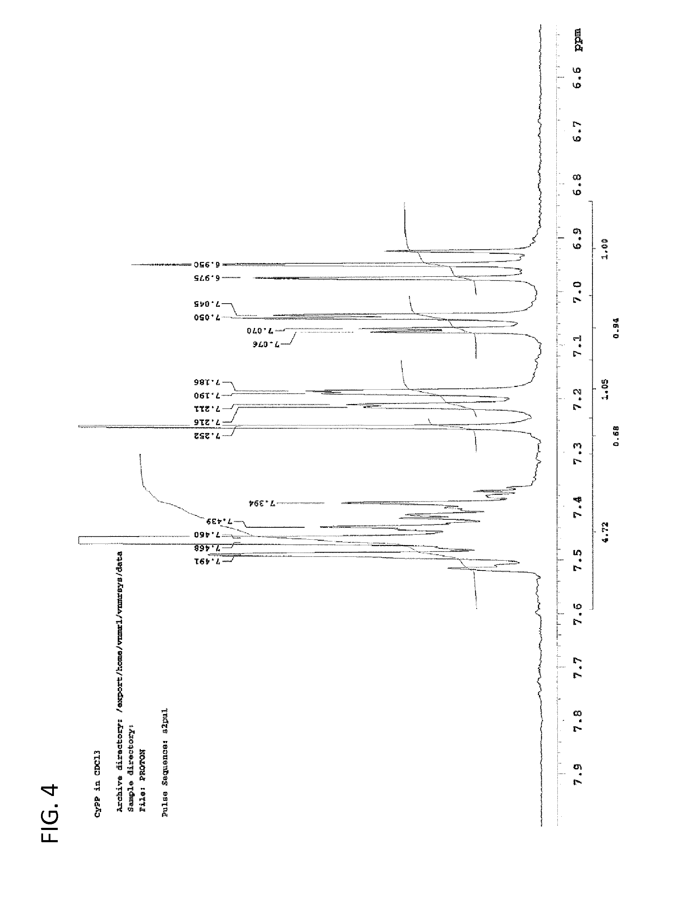

In a nitrogen atmosphere, 2-cyclohexyl-6-phenylphenol (0.34 g, 1.36 mmol, manufactured by Sanko Co., Ltd., or synthesized according to the method described in JP-A 2009-269868 (the same shall apply hereinunder)) was put into a 200-mL reaction flask, followed by purging with nitrogen, and then, toluene (4.9 mL) and triethylaluminium/toluene solution (1.0 mol/L) (0.39 mL, 0.389 mmol) were added thereto in that order, followed by stirring at room temperature for 2 hours, and the solvent was evaporated away to give 0.40 g of a colorless to pale orange, amorphous yellow solid. The resultant solid was dried through concentration under reduced pressure, and the ¹H-NMR spectrum thereof is shown in FIG. 1. FIG. 2 is an enlarged view on the low magnetic field side of the spectrum. The ¹H-NMR spectrum of 2-cyclohexyl-6-phenylphenol is shown in FIG. 3, and FIG. 4 shows an enlarged view on the low-magnetic field side thereof.

234 g of the solid prepared in the above was added to d-citronellal (2.00 g, 13 mmol) cooled at -15 to -10°C, followed by stirring at 0 to 5°C for 1 hour. After the reaction, 2 mL of water and 2 mL of toluene were added, and the organic layer was analyzed through gas chromatography. As a result, the substance conversion was 99.1%, the selectivity of 1-isopulegol was 96.6%, and the ratio of 1-isopulegol to the other isomer was 99.5/0.5.

### (Example 6) Preparation of aluminum catalyst and synthesis of 1-isopulegol

2-Cyclohexyl-6-phenylphenol (344 mg, 1.4 mmol) was put into a 50-mL Schlenk flask, followed by purging with nitrogen, and then, toluene (1.6 mL) and triethylaluminium/toluene solution (1.0 mol/L) (0.4 mL, 0.40 mmol) were added thereto in that order, followed by stirring at room temperature for 2 hours, thereby giving a catalyst solution. The resultant catalyst solution was cooled to -15 to -10°C, and then, d-citronellal (2.00 g, 13 mmol) was dropwise added thereto, followed by stirring at 0 to 5°C for 1 hour. After the reaction, 2 mL of water was added and the organic layer was analyzed through gas chromatography. As a result, the substance conversion was 99.8%, the selectivity of 1-isopulegol was 86.3%, and the ratio of 1-isopulegol to the other isomer was 99.6/0.4.

### (Examples 7 to 12) Synthesis of 1-isopulegol with aluminum catalyst

Various kinds of phenols were used as the hydroxy compound, and the results are shown in Table 1. Regarding the reaction condition, 1.7 mmol of the phenol was put in the 50-mL Schlenk flask in Examples 7 to 11, and 0.87 mmol thereof was put therein in Example 12. After purging with nitrogen, 3 mL in total of toluene as a solvent, and 0.58 mL (0.58 mmol) of triethylaluminium/toluene solution were added in that order, followed by stirring at room temperature for 2 hours, thereby giving a catalyst solution. The catalyst solution was cooled to -10°C, and d-citronellal (3.0 g, 19 mmol) was dropwise added thereto, followed by stirring for 1 hour. After the reaction, 2 mL of water was added and the organic layer was analyzed through gas chromatography.

The phenols in Examples 7 to 12 were produced in the same manner as that for 2-cyclohexyl-6-phenylphenol, according to the method described in JP-A 2009-269868, respectively.

In Table 1, the conversion means the conversion of citronellal, the isopulegol selectivity means the selectivity of the reacted citronellal to isopulegol, the n-isopulegol selectivity means the selectivity of n-isopulegol in the formed isopulegol, and the ester selectivity means the selectivity of citronellal to dimer ester (citronellyl citronellate).

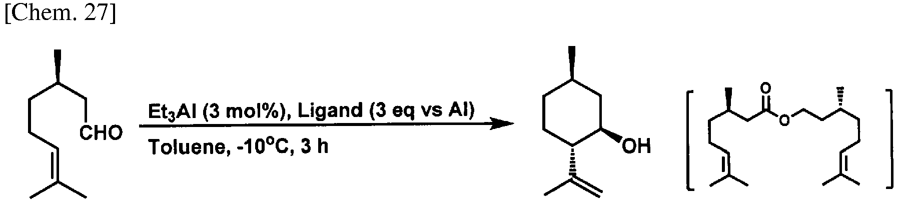

**[Table 1]**

| Example | Ligand | Conversion (%) | Isopulegol Selectivity (%) | n-Selectivity (%) | Ester Selectivity (%) |
|---|---|---|---|---|---|
| 7 | 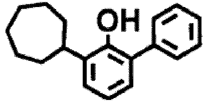 | 99.2 | 97.1 | 99.4 | 2.1 |
| 8 | 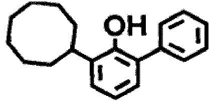 | 98.2 | 93.4 | 99.0 | 4.0 |
| 9 | 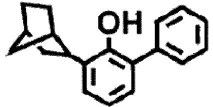 | 99.4 | 98.0 | 99.4 | 0.3 |
| 10 | 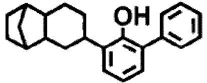 | 98.3 | 89.9 | 98.4 | 5.9 |
| 11 | 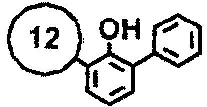 | 99.8 | 95.1 | 98.9 | 1.4 |
| 12 | 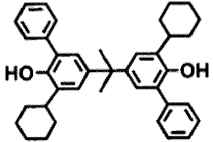 | 99.1 | 95.9 | 99.4 | 1.9 |

### (Examples 13 to 18) Synthesis of 1-isopulegol with aluminum catalyst

2-Cyclohexyl-6-phenylphenol (269 mg, 1,1 mmol) was put in an 50-mL Schlenk flask, followed by purging with nitrogen, and then, toluene (4.7 mL) and triethylaluminum/toluene solution (1.0 mol/L) (0.3 mL, 0.32 mmol) were added thereto in that order, followed by stirring at room temperature for 2 hours, thereby giving a catalyst solution. The resultant catalyst solution was cooled to -15 to -10°C, and then, d-citronellal (5.00 g, 32 mmol), to which 0.5% by weight of the additive shown in Table 2 had been added, was dropwise added thereto, followed by stirring for 3 hours. After the reaction, 2 mL of water was added and the organic layer was analyzed through gas chromatography. The results are shown in Table 2.

In Table 2, the conversion means the conversion of citronellal, the isopulegol selectivity means the selectivity of the reacted citronellal to isopulegol, the n-isopulegol selectivity means the selectivity of n-isopulegol in the formed isopulegol, and the ester selectivity means the selectivity of citronellal to dimer ester (citronellyl citronellate).

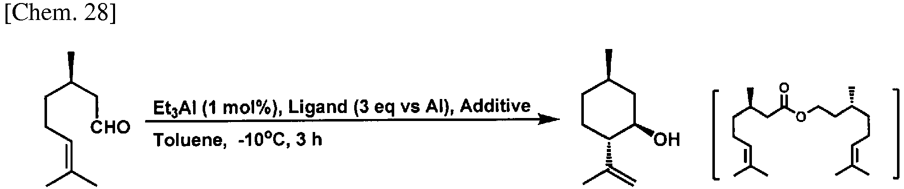

**[Table 2]**

| Example | Additive | Conversion (%) | Isopulegol Selectivity (%) | n-Selectivity (%) | Ester Selectivity (%) |
|---|---|---|---|---|---|
| 13 | acetic anhydride | 99.5 | 96.9 | 99.6 | 0.3 |
| 14 | citronellic acid | 99.5 | 94.6 | 99.5 | 0.6 |
| 15 | monomethyl itaconate | 42.6 | 89.4 | 99.0 | 4.0 |
| 16 | ethyl glyoxylate 40 wt% to 50 wt% polymer/toluene solution | 99.1 | 98.0 | 99.7 | <0.1 |
| 17 | α-pinene oxide | 99.2 | 96.2 | 99.3 | 0.9 |
| 18 | isobutylene oxide | 97.9 | 97.0 | 99.7 | 0.4 |

### (Example 19) Synthesis of 1-isopulegol and ligand recycle

2-Cyclohexyl-6-phenylphenol (10.14 g, 40.2 mmol) was put into a 1-L reaction flask, followed by purging with nitrogen, and 87 mL of toluene and then triethylaluminum toluene solution (1.0 mol/L) (13.0 mL, 13.0 mmol) was added thereto with stirring in a nitrogen atmosphere. After stirred at room temperature for 1 hour, the internal temperature was cooled to -10 to 5°C, and a mixed solution of d-citronellal (100.0 g, 648.3 mmol, optical purity 97.8% e.e.) and ethyl glyoxylate polymer/47 wt% toluene solution (0.33 mL) was dropwise added thereto, followed by stirring overnight at -10 to 5°C. After the reaction, toluene was removed under reduced pressure, and the solution was processed for simple distillation (bath temperature 85°C, degree or reduced pressure 0.5 mmHg, top temperature 58 to 61°C), thereby giving 94.5 of the intended 1-n-isopulegol. As a result of analysis through gas chromatography, the purity was 97.8%, and the ratio of 1-n-isopulegol to the other isomer was 99.6/0.4. The distillation residue was dissolved in toluene, washed with diluted hydrochloric acid, and then the oily layer was concentrated under reduced pressure, thereby giving a viscous liquid. This was processed through simple distillation (101 to 120°C, 0.04 kPa) to give 8.02 g of 2-cyclohexyl-6-phenylphenol. As a result of analysis through gas chromatography, the purity was 98.2 %.

The obtained 2-cyclohexyl-6-phenylphenol (324 mg, 1.284 mmol) was put into a 50-mL Schlenk, followed by purging with nitrogen, and toluene (2.6 mL) and triethylaluminium/toluene solution (1.0 mol/L) (0.39 mL, 0.39 mmol) were added with stirring in the nitrogen atmosphere. After stirred for 1 hour at room temperature, the internal temperature was cooled to -10 to 5°C, and a mixed solution of d-citronellal (3.00 g, 19.45 mmol, optical purity 97.8% e.e.) and ethyl glyoxylate polymer/47 wt% toluene solution (0.27 µL) was dropwise added thereto, followed by stirring overnight at -10 to 5°C. After the reaction, 3 mL of water was added to the solution, and the organic layer was analyzed through gas chromatography. As a result, the substance conversion was 99.7%, the selectivity of isopulegol was 98.0%, the ratio of 1-n-isopulegol to the other isomer was 99.6/0.4.

### (Example 20) Synthesis of 1-isopulegol

2-Cyclohexyl-6-phenylphenol (3.56 g, 14.7 mmol) was put into a 1-L reaction flask, followed by purging with nitrogen, and toluene (96 mL) and then triethylaluminum/toluene solution (1.0 mol/L) (4.54 mL, 4.54 mmol) were added thereto with stirring in the nitrogen atmosphere. After stirred at room temperature for 1 hour, the internal temperature was cooled to -10 to 5°c, and a mixed solution of d-citronella (100.0 g, 648.3 mmol, optical purity 99% e.e.) obtained in Example 3 and ethyl glyoxylate polymer/47 wt.% toluene solution (0.33 mL) was dropwise added thereto, followed by stirring overnight at -10 to 5°C. After the reaction, water was added to the solution sample, and the organic layer was analyzed through gas chromatography. As a result, the substance conversion was 99.5%, the selectivity of isopulegol was 98.2% and the optical purity was 99% e.e. The ratio of 1-n-isopulegol to the other isomer was 99.7/0.3. After toluene evaporation under reduced pressure, the simple distillation was performed (57 to 61°C, 0.053 to 0.067 kPa), thereby giving 95.8 g of the intended 1-n-isopulegol. As a result of gas chromatography, the purity was 97.6%, and the ratio of 1-n-isopulegol to the other isomer was 99.7/0.3. The reaction was repeated twice.

### (Comparative Examples 1 to 18) Synthesis of 1-isopulegol with aluminum catalyst

The results of using various kinds of phenols as the hydroxy compound are shown in Table 3 and Table 4. Regarding the reaction condition, a predetermined amount (2.0 mmol) of the phenol was put into a 50-mL Schlenk flask, followed by purging with nitrogen, and as a solvent, 3 mL in total of toluene and 0.58 mL (0.58 mmol) of triethylaluminium/toluene solution were added thereto in that order, followed by stirring for 2 hours at room temperature, thereby preparing a catalyst solution. The catalyst solution was cooled down to a predetermined temperature, and then, d-citronellal (3.0 g, 19 mmol) was dropwise added thereto, followed by stirring for 1 hour at a predetermined temperature. After the reaction, 2 mL of water was added and the organic layer was analyzed through gas chromatography.

The phenols in Comparative Examples 1 to 15, 17 and 18 were a product of Aldrich , and the phenol in Comparative Example 16 was a product of Bepharm.

In Table 3 and Table 4, the conversion means the citronellal conversion, the isopulegol selectivity means the selectivity of the reacted citronellal to isopulegol, the n-isopulegol selectivity means the selectivity of n-isopulegol in the formed isopulegol, and the ester selectivity means the selectivity of citronellal to dimer ester (citronellyl citronellate).

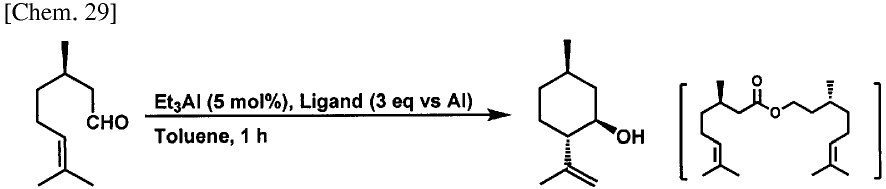

**[Table 3]**

| Comparative Example | Ligand | Temperature (°C) | Conversion (%) | Isopulegol Selectivity (%) | n-Selectivity (%) | Ester Selectivity (%) |
|---|---|---|---|---|---|---|
| 1 | 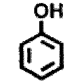 | 0 | 54.9 | 12.9 | 92.3 | 81 |
| 2 | 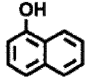 | 0 | 33.8 | 68.3 | 74.7 | 25.9 |
| 3 | 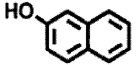 | 0 | 29.8 | 37.7 | 87.7 | 52.1 |
| 4 | 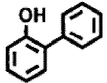 | 0 | 97 | 21.9 | 90.9 | 75.2 |
| 5 | 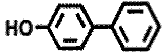 | 0 | 52.7 | 19.2 | 92.3 | 74.9 |
| 6 | 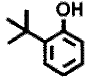 | 0 | 74.3 | 10.5 | 89.5 | 70.9 |
| 7 | 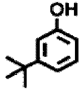 | 25 | 11.7 | 69.7 | 87.7 | 11.5 |
| 8 | 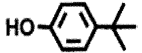 | 25 | 10.5 | 91.6 | 78.6 | 4.7 |
| 9 | 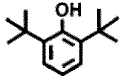 | 0 | 85.5 | trace | - | 90.9 |

**[Table 4]**

| Comparative Example | Ligand | Temperature (°C) | Conversion (%) | Isopulegol Selectivity (%) | n-Selectivity (%) | Ester Selectivity (%) |
|---|---|---|---|---|---|---|
| 10 | 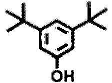 | 25 | 35.5 | 19.4 | 93.2 | 62.8 |
| 11 | 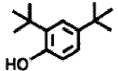 | 0 | 61.6 | 4.6 | 87.1 | 92.1 |
| 12 | 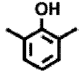 | 0 | 88.4 | 18.7 | 85.3 | 74.4 |
| 13 | 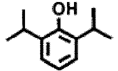 | 0 | 76.7 | 9.4 | 91.8 | 87.3 |
| 14 | 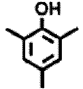 | 0 | 95.5 | 6.1 | 88.8 | 84.6 |
| 15 | 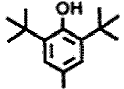 | 0 | 87 | trace | - | 89.9 |
| 16 | 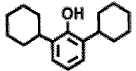 | -10 | 3.2 | 80.1 | 80.4 | 0.0 |
| 17 | 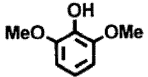 | 25 | trace | - | - | - |
| 18 | 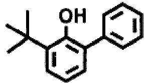 | -10 | 97.6 | 0.86 | 87.8 | 85.2 |

### (Example 21) Hydrogenation reaction of high-purity 1-isopulegol, synthesis of high-purity 1-menthol

In a nitrogen atmosphere, 100.0 g (0.65 mol) of 1-isopulegol obtained in Example 20 and 0.4 g of Raney nickel were put into a 500-mL autoclave, and hydrogenation was carried out under a hydrogen pressure of 2.5 MPa at 70°C for 10 hours. The reaction liquid was filtered and distilled (bp 212°C), thereby giving 94.0 g (0.60 mol, 99% e.e.) of 1-menthol.

While the invention has been described in detail and with reference to specific embodiments thereof, it will be apparent to one skilled in the art that various changes and modifications can be made therein without departing from the spirit and scope thereof. The present application is based on Japanese Patent Application No. 2012-251301 filed on November 15, 2012 and Japanese Patent Application No. 2013-044065 filed on March 6, 2013, the contents of which are incorporated herein by reference.

### Industrial Applicability

The optically active citronellal used in the present invention can be produced through asymmetric isomerization of geraniol and/or nerol using an optically active ruthenium catalyst.

By merely mixing an alkylaluminum compound and a specific alcohol, the cyclization reaction catalyst for citronellal for use in the present invention can cyclize citronellal and optically active citronellal, thereby giving a highly n-selective isopulegol and an optically active isopulegol.

The resultant optically active isopulegol can be processed for cryogenic crystallization, thereby giving a high-purity optically active isopulegol, and the resultant high-purity optically active isopulegol or an optically active isopulegol not processed for cryogenic crystallization can be hydrogenated using an ordinary hydrogenation catalyst for carbon-carbon double bond, thereby giving an optically active menthol.

As described above, the production method for an optically active menthol in the present invention includes an extremely short steps, and all the steps therein are catalytic reaction steps. Accordingly, the production method releases few environment-polluting wastes and reduces the production expenses.

## Claims

1. A method for producing an optically active isopulegol represented by the following general formula (3), the method comprising a step of asymmetrically isomerizing a compound represented by the following general formula (1) in the presence of a ruthenium catalyst and a base, thereby giving an optically active citronellal represented by the following general formula (2) and a step of selectively cyclizing the optically active citronellal represented by the general formula (2) in the presence of an aluminum catalyst,
wherein the aluminum catalyst is one obtained by reacting a hydroxy compound represented by the following general formula (5) or a hydroxy compound represented by the following general formula (6) and at least one aluminum compound selected from an alkylaluminum compound represented by the following general formula (7), a hydride aluminum compound represented by the following general formula (8), a linear aluminoxane represented by the following general formula (9) and a cyclic aluminoxane represented by the following general formula (10): 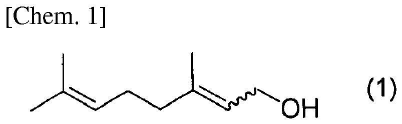 wherein the wavy line represents an (E) form and/or (Z) form of the double bond, 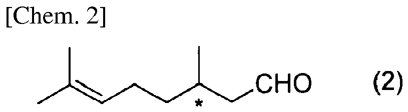 wherein * indicates an asymmetric carbon atom, 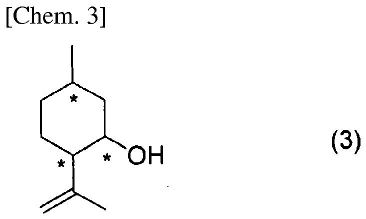 wherein * indicates an asymmetric carbon atom, 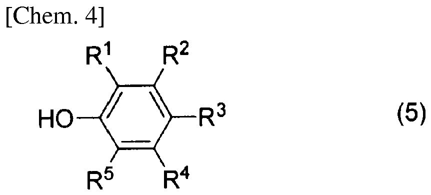 wherein R¹ and R⁵ each independently represents an aryl group having from 6 to 15 carbon atoms, which may have a substituent, a heteroaryl group having from 4 to 15 carbon atoms, which may have a substituent, or a cyclic alkyl group having from 3 to 15 carbon atoms, which may have a substituent; R², R³ and R⁴ each independently represents a hydrogen atom, an alkyl group having from 1 to 8 carbon atoms, an alkoxy group having from 1 to 8 carbon atoms, an aryl group having from 6 to 15 carbon atoms, which may have a substituent, a perfluoroalkyl group having from 1 to 4 carbon atoms, an aralkyl group having from 7 to 12 carbon atoms, a halogen atom, an organosilyl group, a dialkylamino group having from 1 to 4 carbon atoms, a thioalkoxy group having from 1 to 4 carbon atoms, a nitro group, or a polymer chain, 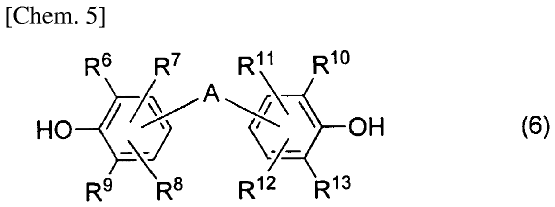 wherein R⁶, R⁹, R¹⁰ and R¹³ each independently represents an aryl group having from 6 to 15 carbon atoms, which may have a substituent, a heteroaryl group having from 4 to 15 carbon atoms, which may have a substituent, or a cyclic alkyl group having from 3 to 15 carbon atoms, which may have a substituent; R⁷, R⁸, R¹¹ and R¹² each independently represents a hydrogen atom, an alkyl group having from 1 to 8 carbon atoms, an alkoxy group having from 1 to 8 carbon atoms, an aryl group having from 6 to 15 carbon atoms, which may have a substituent, a perfluoroalkyl group having from 1 to 4 carbon atoms, an aralkyl group having from 7 to 12 carbon atoms, a halogen atom, an organosilyl group, a dialkylamino group having from 1 to 4 carbon atoms, a thioalkoxy group having from 1 to 4 carbon atoms, a nitro group, or a polymer chain,
A represents (i) a single bond, (ii) a linear, branched or cyclic alkylene group having from 1 to 25 carbon atoms, which may have one or more of a substituent and an unsaturated bond, (iii) an arylene group having from 6 to 15 carbon atoms, which may have a substituent, (iv) a heteroarylene group having from 2 to 15 carbon atoms, which may have a substituent, or (v) a functional group or a hetero element, which is selected from the group consisting of -O-, -S-, -N(R¹⁴)-, -S(O)-, -C(O)-, -S(O)₂-, -P(R¹⁴)-, -(R¹⁴)P(O)- and -Si(R¹⁵R¹⁶)-; R¹⁴ to R¹⁶ each independently represents an alkyl group having from 1 to 6 carbon atoms, a cyclic alkyl group having from 5 to 8 carbon atoms, an aralkyl group having from 7 to 12 carbon atoms, which may have a substituent, or an aryl group having from 6 to 10 carbon atoms, which may have a substituent,
AlHₖ(Lg)₃₋ₖ (7)
wherein Al represents aluminum, Lg represents a branched or linear alkyl group having from 1 to 8 carbon atoms, a cyclic alkyl group having from 5 to 8 carbon atoms, or an aralkyl group having from 7 to 12 carbon atoms, which may have a substituent; and k indicates an integer of from 0 to 3,
MAlH₄ (8)
wherein Al represents aluminum, M represents lithium, sodium or potassium, 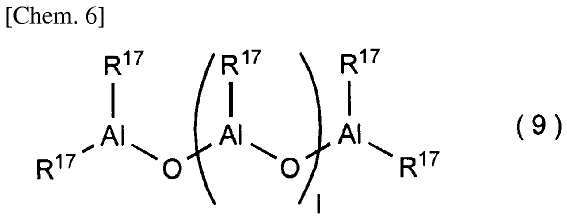 wherein Al represents aluminum, R¹⁷ represents a branched or linear alkyl group having from 1 to 8 carbon atoms, a cyclic alkyl group having from 5 to 8 carbon atoms, or an aralkyl group having from 7 to 12 carbon atoms, which may have a substituent, and a plurality of R¹⁷ may be the same or different; and 1 indicates an integer of from 0 to 40, 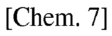 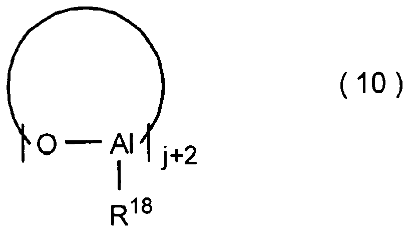 wherein Al represents aluminum, R¹⁸ represents a branched or linear alkyl group having from 1 to 8 carbon atoms, a cyclic alkyl group having from 5 to 8 carbon atoms, or an aralkyl group having from 7 to 12 carbon atoms, which may have a substituent; and j indicates an integer of from 0 to 40.

2. The method for producing an optically active isopulegol according to claim 1, wherein the hydroxy compound represented by the general formula (5) is a hydroxy compound represented by the following general formula (5-a): 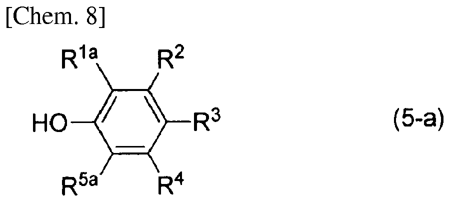 wherein R^{1a} represents an aryl group having from 6 to 15 carbon atoms, which may have a substituent, or a heteroaryl group having from 4 to 15 carbon atoms, which may have a substituent; R^{5a} represents a cyclic alkyl group having from 5 to 15 carbon atoms, which may have a substituent; R², R³ and R⁴ each independently represents a hydrogen atom, an alkyl group having from 1 to 8 carbon atoms, an alkoxy group having from 1 to 8 carbon atoms, an aryl group having from 6 to 15 carbon atoms, which may have a substituent, a perfluoroalkyl group having from 1 to 4 carbon atoms, an aralkyl group having from 7 to 12 carbon atoms, a halogen atom, an organosilyl group, a dialkylamino group having from 1 to 4 carbon atoms, a thioalkoxy group having from 1 to 4 carbon atoms, a nitro group, or a polymer chain.

3. The method for producing an optically active isopulegol according to claim 1, wherein the hydroxy compound represented by the general formula (6) is a hydroxy compound represented by the following general formula (6-a): 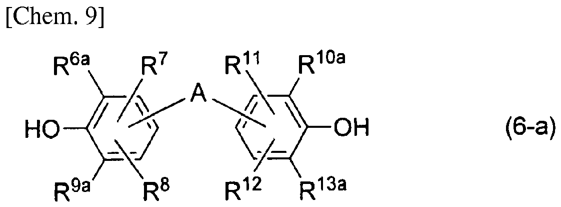 wherein R^{6a} and R^{10a} each independently represents an aryl group having from 6 to 15 carbon atoms, which may have a substituent, or a heteroaryl group having from 4 to 15 carbon atoms, which may have a substituent; R^{9a} and R^{13a} each represents a cyclic alkyl group having from 5 to 15 carbon atoms, which may have a substituent; R⁷, R⁸, R¹¹ and R¹² each independently represents a hydrogen atom, an alkyl group having from 1 to 8 carbon atoms, an alkoxy group having from 1 to 8 carbon atoms, an aryl group having from 6 to 15 carbon atoms, which may have a substituent, a perfluoroalkyl group having from 1 to 4 carbon atoms, an aralkyl group having from 7 to 12 carbon atoms, a halogen atom, an organosilyl group, a dialkylamino group having from 1 to 4 carbon atoms, a thioalkoxy group having from 1 to 4 carbon atoms, a nitro group, or a polymer chain,
A represents (i) a single bond, (ii) a linear, branched or cyclic alkylene group having from 1 to 25 carbon atoms, which may have one or more of a substituent and an unsaturated bond, (iii) an arylene group having from 6 to 15 carbon atoms, which may have a substituent, (iv) a heteroarylene group having from 2 to 15 carbon atoms, which may have a substituent, or (v) a functional group or a hetero element, which is selected from the group consisting of -O-, -S-, -N(R¹⁴)-, -S(O)-, -C(O)-, -S(O)₂-, -P(R¹⁴)-, -(R¹⁴)P(O)- and -Si(R¹⁵R¹⁶)-; R¹⁴ to R¹⁶ each independently represents an alkyl group having from 1 to 6 carbon atoms, a cyclic alkyl group having from 5 to 8 carbon atoms, an aralkyl group having from 7 to 12 carbon atoms, which may have a substituent, or an aryl group having from 6 to 10 carbon atoms, which may have a substituent.

4. The method for producing an optically active isopulegol according to any one of claims 1 to 3, wherein the cyclization reaction is carried out in the presence of at least one compound of the following compounds I and II:
I. at least one acid,
II. at least one compound selected from the group containing an aldehyde except citronellal, an acid anhydride, a ketone, an acid halide, an epoxy compound and a vinyl ether.

5. The method for producing an optically active isopulegol according to any one of claims 1 to 4, wherein the ruthenium catalyst is a ruthenium compound represented by the following general formula (11):
[RuₘLₙWₚU_{q}]ᵣZₛ (11)
wherein L represents an optically active phosphine ligand; W represents a hydrogen atom, a halogen atom, an acyloxy group, an aryl group, a diene or an anion; U represents a hydrogen atom, a halogen atom, an acyloxy group, an aryl group, a diene, an anion or a ligand except L; Z represents an anion, an amine or an optically active nitrogen-containing compound; m, n and r each independently indicate an integer of from 1 to 5; p, q and s each independently indicate an integer of from 0 to 5; and p + q + s is 1 or more.

6. The method for producing an optically active isopulegol according to any one of claims 1 to 5, wherein the base is a salt of an alkali metal or alkaline earth metal, or a quaternary ammonium salt.

7. A method for producing an optically active menthol, comprising a step of preparing an optically active isopulegol according to the method as described in any one of claims 1 to 6, and a step of hydrogenating the optically active isopulegol prepared.

8. A method for producing an optically active menthol, comprising the following steps:
A-1) preparing an optically active citronellal through asymmetric isomerization of geraniol or nerol,
B-1) preparing an optically active isopulegol through cyclization reaction of the optically active citronellal with an acidic catalyst, and
C-1) hydrogenating the optically active isopulegol, thereby giving the optically active menthol.

9. A method for producing an optically active menthol, comprising the following steps:
A-2) preparing an optically active citronellal through asymmetric isomerization of geraniol or nerol,
B-2) preparing an optically active isopulegol through cyclization reaction of the optically active citronellal with an acidic catalyst,
D-2) preparing an isopulegol having a further higher purity through cryogenic recrystallization of the optically active isopulegol, and
E-2) hydrogenating the optically active isopulegol prepared in the step D-2, thereby giving the optically active menthol.

10. A method for producing an optically active menthol, comprising the following steps:
A-3) preparing d-citronellal through asymmetric isomerization of geraniol or nerol,
B-3) preparing 1-isopulegol through cyclization reaction of the d-citronellal with an acidic catalyst, and
C-3) hydrogenating the 1-isopulegol, thereby giving 1-menthol.

11. A method for producing an optically active menthol, comprising the following steps:
A-4) preparing d-citronellal through asymmetric isomerization of geraniol or nerol,
B-4) preparing 1-isopulegol through cyclization reaction of the d-citronellal with an acidic catalyst,
D-4) preparing 1-isopulegol having a further higher purity through cryogenic recrystallization of the 1-isopulegol, and
E-4) hydrogenating the 1-isopulegol prepared in the step D-4, thereby giving 1-menthol.
